# EUROPEAN PATENT SPECIFICATION

(11) **EP 1 794 143 B1**
(45) Date of publication and mention of the grant of the patent: **09.07.2008**
(21) Application number: 05786700.4
(22) Date of filing: 22.08.2005
(51) Int. Cl.: C07D 333/58, C07D 307/81, C07D 209/14, A61K 31/381, A61K 31/343, A61K 31/404, A61P 25/08

(54) **NOVEL BENZO-FUSED HETEROARYL SULFAMIDE DERIVATIVES USEFUL AS ANTICONVULSANT AGENTS**
ALS ANTIKONVULSIVE MITTEL GEEIGNETE NEUE BENZOKONDENSIERTE HETEROARYLSULFAMIDDERIVATE
NOUVEAUX COMPOSÉS POLYAROMATIQUES DE TYPE HÉTÉROARYLSULFAMIDE POUVANT ÊTRE EMPLOYÉS EN TANT QU'AGENTS ANTICONVULSIVANTS

(30) Priority: 24.08.2004 US 604134 P
(43) Date of publication of application: 13.06.2007
(73) Proprietor: Janssen Pharmaceutica NV, 2340 Beerse (BE); Parker, Michael H., Chalfot PA 18914 (US)
(72) Inventor: PARKER, Michael H., Chalfot, PA 18914 (US); REITZ, Allen B., Lansdale, PA 19446 (US); MARYANOFF, Bruce E., Forest Grove, PA 18922 (US)
(74) Representative: Kirsch, Susan Edith
(86) International application number: PCT/US2005/029814
(87) International publication number: WO 2006/023861

(56) References cited:
- EP-A- 0 483 881
- WO-A-97/19682
- WO-A-02/096424

## Description

The present invention is directed to novel benzo-fused heteroaryl sulfamide derivatives, pharmaceutical compositions containing them and their use in the treatment of epilepsy and related disorders.

Epilepsy describes a condition in which a person has recurrent seizures due to a chronic, underlying process. Epilepsy refers to a clinical phenomenon rather than a single disease entity, since there are many forms and causes of epilepsy. Using a definition of epilepsy as two or more unprovoked seizures, the incidence of epilepsy is estimated at approximately 0.3 to 0.5 percent in different populations throughout the world, with the prevalence of epilepsy estimated at 5 to 10 people per 1000.

An essential step in the evaluation and management of a patient with a seizure is to determine the type of seizure that has occurred. The main characteristic that distinguishes the different categories of seizures is whether the seizure activity is partial (synonymous with focal) or generalized.

Partial seizures are those in which the seizure activity is restricted to discrete areas of the cerebral cortex. If consciousness is fully preserved during the seizure, the clinical manifestations are considered relatively simple and the seizure is termed a simple-partial seizure. If consciousness is impaired, the seizure is termed a complex-partial seizure. An important additional subgroup comprises those seizures that begin as partial seizures and then spread diffusely throughout the cortex, which are known as partial seizures with secondary generalization.

Generalized seizures involve diffuse regions of the brain simultaneously in a bilaterally symmetric fashion. Absence or petit mal seizures are characterized by sudden, brief lapses of consciousness without loss of postural control. Atypical absence seizures typically include a longer duration in the lapse of consciousness, less abrupt onset and cessation, and more obvious motor signs that may include focal or lateralizing features. Generalized Tonic-clonic or grand mal seizures, the main type of generalized seizures, are characterized by abrupt onset, without warning. The initial phase of the seizure is usually tonic contraction of muscles, impaired respiration, a marked enhancement of sympathetic tone leading to increased heart rate, blood pressure, and pupillary size. After 10-20 s, the tonic phase of the seizure typically evolves into the clonic phase, produced by the superimposition of periods of muscle relaxation on the tonic muscle contraction. The periods of relaxation progressively increase until the end of the ictal phase, which usually lasts no more than 1 min. The postictal phase is characterized by unresponsiveness, muscular flaccidity, and excessive salivation that can cause stridorous breathing and partial airway obstruction. Atonic seizures are characterized by sudden loss of postural muscle tone lasting 1-2 s. Consciousness is briefly impaired, but there is usually no postictal confusion. Myoclonic seizures are characterized by a sudden and brief muscle contraction that may involve one part of the body or the entire body.

Carbonic anhydrase inhibitors (CAls) have been widely used in medicine, mainly as antiglaucoma and antisecretory drugs or diuretic agents, and are valuable compounds. However, systemic antiglaucoma agents (such as acetazolamide) possess potentially unwanted side-effects including paresthesias, nephro6thiasis and weight loss. Topiramate is a well known anticonvulsant drug that possesses single digit micromolar carbonic anhydrase inhibition, which is suspected as the cause of paresthesias noted by some patients taking topiramate.

EP-A-0483881 describes 3-amido and 3-sulfamido-indolyl NMDA antagonists and their use in the treatment of a number of disease states.

WO-A-97/19682 describes aryl salfonamide and sulfamide compounds which are said to bind selectively to and inhibit the activity of the human Y5 receptor. Also described are the use of those compounds for the treatment of feeding disorders such as obesity, anorexia nervosa, bulimia nervosa, and abnormal conditions such as sexual/reproductive disorders, depression, epileptic seizure, hypertension, cerebral hemorrhage, congestive heart failure or sleep disturbances and for the treatment of any disease in which antagonism of a Y5 receptor may be useful

WO-A-02/096424 describes a method for preventing or treating epileptogenesis-associated diseases comprising the administration of a beta -heterocyclic- beta - aminoacid to a subject. The disease can be, for example, head trauma, pain, stroke, anxiety, schizophrenia, psychosis, cerebral ischemia, Huntington's chorea, motor neuron disease, Alzheimer's disease, dementia or epilepsy,

There remains a need to provide an effective treatment for epilepsy and related disorders, and preferably treatment which does not have the associated side-effects attributable to carbonic anhydrase inhibition.

### SUMMARY OF THE INVENTION

The present invention is directed to a compound of formula (I) 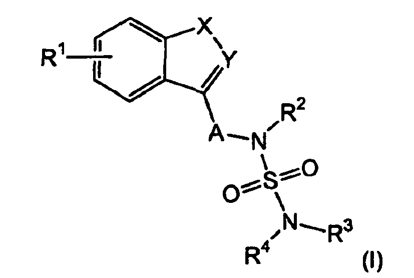 wherein
R¹ is selected from the group consisting of hydrogen, halogen, hydroxy, methoxy, trifluoromethyl, nitro and cyano;
X-Y is selected from the group consisting of -S-CH-, -S-C(CH₃)-, -O-CH-, -O-C(CH₃)-, -N(CH₃)-CH- and -CH=CH-CH-;
A is selected from the group consisting of -CH₂- and -CH(CH₃)-;
R² is selected from the group consisting of hydrogen and methyl;
R³ and R⁴ are each independently selected from the group consisting of hydrogen and C₁₋₄alkyl;
alternatively, R³ and R⁴ are taken together with the nitrogen atom to which they are bound to form a 5 to 7 membered, saturated, partially unsaturated or aromatic ring structure, optionally containing one to three additional heteroatoms independently selected from the group consisting of O, N and S;
or a pharmaceutically acceptable salt thereof.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

Another example of the invention is the use of any of the compounds described herein in the preparation of a medicament for treating epilepsy or a related disorder selected from seizures, essential tremor or restless limb syndrome, in a subject in need thereof.

### DETAILED DESCRIPTION OF THE INVENTION

The present invention is directed to compounds of formula (I) 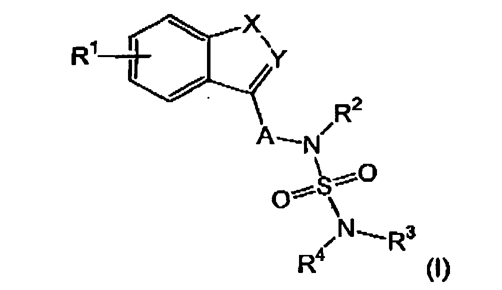 wherein R¹, R², R³, R⁴, -X-Y- and A are as herein defined. The compounds of formula (I) are useful for treating epilepsy and related disorders.

In an embodiment, the present invention is directed to a compound of formula (I) 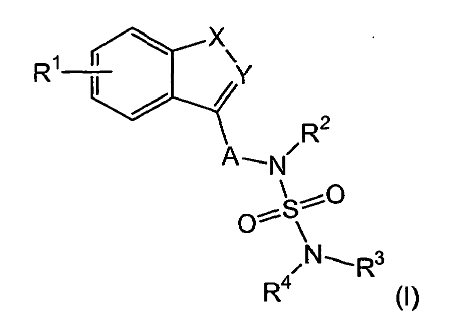 wherein
R¹ is selected from the group consisting of hydrogen, halogen, hydroxy, methoxy, trifluoromethyl, nitro and cyano;
X-Y is selected from the group consisting of -S-CH-, -S-C(CH₃)-, -O-CH-, -O-C(CH₃)-, -N(CH₃)-CH- and -CH=CH-CH-;
A is selected from the group consisting of-CH₂- and -CH(CH₃)-;
R² is selected from the group consisting of hydrogen and methyl;
R³ and R⁴ are each independently selected from the group consisting of hydrogen and methyl;
alternatively, R³ and R⁴ are taken together with the nitrogen atom to which they are bound to form a 5 to 7 membered, saturated, partially unsaturated or aromatic ring structure, optionally containing one to two additional heteroatoms independently selected from the group consisting of O, N and S;
or a pharmaceutically acceptable salt thereof.

In an embodiment of the present invention are compounds of formula (I) wherein
R¹ is selected from the group consisting of hydrogen and halogen;
X-Y is selected from the group consisting of -S-CH-, -S-C(CH₃)-, -O-CH-, -O-C(CH₃)-, -N(CH₃)-CH- and -CH=CH-CH-;
A is selected from the group consisting of -CH₂- and -CH(CH₃)-;
R² is selected from the group consisting of hydrogen and methyl;
R³ and R⁴ are each independently selected from the group consisting of hydrogen and methyl;
and pharmaceutically acceptable salts thereof.

In another embodiment of the present invention are compounds of formula (I) wherein
R¹ is selected from the group consisting of hydrogen and halogen; wherein the halogen is bound at the 4-, 5- or 7-position;
X-Y is selected from the groups consisting of -O-CH-, -O-C(CH₃)-, -S-CH-, -S-C(CH₃)-, -N(CH₃)-CH- and -CH=CH-CH-;
A is selected from the group consisting of -CH₂- and -CH(CH₃)-;
R² is hydrogen;
R³ and R⁴ are each hydrogen;
and pharmaceutically acceptable salts thereof.

In another embodiment of the present invention are compounds of formula (I) wherein
R¹ is hydrogen;
X-Y is selected from the groups consisting of-O-CH-, -O-C(CH₃)-, -S-CH-, -S-C(CH₃)-, -N(CH₃)-CH- and -CH=CH-CH-;
A is selected from the group consisting of -CH₂- and -CH(CH₃)-;
R² is hydrogen;
R³ and R⁴ are each hydrogen;
and pharmaceutically acceptable salts thereof.

In another embodiment of the present invention are compounds of formula (I) wherein
R¹ is selected from the group consisting of hydrogen halogen, hydroxy, methoxy, trifluoromethyl, nitro and cyano; preferably, R¹ is selected from the group consisting of hydrogen and halogen; more preferably, R¹ is selected from the group consisting of hydrogen and halogen, wherein the halogen is bound at the 4-, 5- or 7-position;
X-Y is -S-CH-;
A is selected from the group consisting of -CH₂- and -CH(CH₃)-;
R² is selected from the group consisting of hydrogen and methyl; preferably, R² is hydrogen;
R³ and R⁴ are each independently selected from the group consisting of hydrogen and halogen; preferably, R³ and R⁴ are each hydrogen;
and pharmaceutically acceptable salts thereof.

In an embodiment of the present invention R¹ is selected from the group consisting of hydrogen, chloro, fluoro and bromo. In another embodiment of the present invention, the R¹ group is other than hydrogen and bound at the 4-, 5- or 7-position, preferably at the 5-position. In yet another embodiment of the present invention, the R¹ group is other than hydrogen and bound at the 5-, 6- or 8-position, preferably at the 6-position. In yet another embodiment of the present invention, R¹ is selected from the group consisting of hydrogen and halogen. In yet another embodiment of the present invention, R¹ is selected from the group consisting of hydroxy and methoxy. In yet another embodiment of the present invention, R¹ is selected from the group consisting of hydrogen, halogen and trifluoromethyl. In yet another embodiment of the present invention, R¹ is selected from the group consisting of hydrogen, halogen, trifluoromethyl, cyano and nitro. In yet another embodiment of the present invention, R¹ is selected from the group consisting of hydrogen, halogen, trifluoromethyl and cyano. In yet another embodiment of the present invention, R¹ is selected from the group consisting of trifluoromethyl and cyano. In yet another embodiment of the present invention, R1 is selected from the group consisting of hydrogen, 4-bromo, 5-chloro, 5-fluoro, 5-bromo, 5-trifluoromethyl-5-cyano and 7-cyano.

In an embodiment of the present invention R² is hydrogen. In another embodiment of the present invention R³ and R⁴ are each hydrogen. In yet another embodiment of the present invention R² is hydrogen, R³ is hydrogen and R⁴ is hydrogen.

In an embodiment of the present invention, R³ and R⁴ are each independently selected from the group consisting of hydrogen and C₁₋₄alkyl. In another embodiment of the present invention, R³ and R⁴ are taken together with the nitrogen atom to which they are bound to form a 5 to 7 membered, saturated, partially unsaturated or aromatic ring structure, optionally containing one to two additional heteroatoms independently selected from the group consisting of O, N and S.

In an embodiment of the present invention, R³ and R⁴ are each independently selected from the group consisting of hydrogen, methyl and ethyl. In another embodiment of the present invention, R³ and R⁴ are each independently selected from the group consisting of hydrogen and methyl. In yet another embodiment of the present invention, R³ and R⁴ are each independently selected from the group consisting of hydrogen and ethyl. In yet another embodiment of the present invention, R³ is hydrogen and R⁴ is ethyl.

In an embodiment of the present invention R³ and R⁴ are taken together with the nitrogen atom to which they are bound to form a 5 to 7 membered, saturated, partially unsaturated or aromatic ring structure, optionally containing one to two additional heteroatoms independently selected from the group consisting of O, S and N. In another embodiment of the present invention R³ and R⁴ are taken together with the nitrogen atom to which they are bound to form a 5 to 7 membered saturated ring structure, optionally containing one to two additional heteroatoms independently selected from the group consisting of O, S and N. In another embodiment of the present invention R³ and R⁴ are taken together with the nitrogen atom to which they are bound to form a 5 to 7 membered aromatic ring structure, optionally containing one to two additional heteroatoms independently selected from the group consisting of O, S and N.

Preferably, R³ and R⁴ are taken together with the nitrogen atom to which they are bound to form a 5 to 6 membered saturated, partially unsaturated or aromatic ring structure, optionally containing one to two additional heteroatoms independently selected from the group consisting of O, S and N. More preferably, R³ and R⁴ are taken together with the nitrogen atom to which they are bound to form a 6 membered saturated, partially unsaturated or aromatic ring structure, optionally containing one to two additional heteroatoms independently selected from the group consisting of O, S and N.

Preferably, R³ and R⁴ are taken together with the nitrogen atom to which they are bound to form a 5 to 7 (more preferably 5 to 6) membered saturated or aromatic ring structure, optionally containing one to two (preferably one) additional heteroatoms independently selected from the group consisting of O, S and N (preferably O or N, more preferably N).

In another embodiment of the present invention, R³ and R⁴ are taken together with the nitrogen atom to which they are bound to form a 5 to 6 membered saturated or aromatic ring structure, optionally containing one to two (preferably one) additional heteroatoms independently selected from the group consisting of O, S and N (preferably O or N, more preferably, N).

Preferably, the 5 to 7 membered saturated, partially unsaturated or aromatic ring structure contains 0 to 1 additional heteroatoms independently selected from the group consisting of O, S and N. Preferably, the heteroatom is independently selected from the group consisting of O and N, more preferably, the heteroatom is N.

Suitable examples of the 5 to 7 membered, saturated, partially unsaturated or aromatic ring structures which optionally contain one to two additional heteroatoms independently selected from the group consisting of O, S and N include, but are not limited to pyrrolyl, pyrrolidinyl, pyrrolinyl, morpholinyl, piperidinyl, piperazinyl, imidazolyl, pyrazolyl, pyridyl, imidazolyl, thiomorpholinyl, pyrazinyl, triazinyl, azepinyl, and the like. Preferred 5 to 7 membered, saturated, partially unsaturated or aromatic ring structures which optional containing one to two additional heteroatoms independently selected from the group consisting of O, S and N include, but are not limited, to imidazolyl, pyrrolidinyl, piperidinyl and morpholinyl.

In an embodiment of the present invention A is -CH₂-.

In an embodiment of the present invention X-Y is selected from the group consisting of -S-CH-, -O-CH-, -O-C(CH₃)-, -N(CH₃)-CH- and -CH=CH-CH-. In another embodiment of the present invention X-Y is selected from the group consisting of -S-CH-, -O-CH-, -O-C(CH₃)- and -CH=CH-CH-. In yet another embodiment of the present invention X-Y is selected form the group consisting of-S-CH-, -O-CH-, -O-C(CH₃)- and -N(CH₃)-CH-. In yet another embodiment of the present invention X-Y is selected from the group consisting of-S-CH-, -O-CH-, -N(CH₃)-CH- and -CH=CH-CH-. In yet another embodiment of the present invention X-Y is selected from the group consisting of -S-CH-, -O-CH- and -CH=CH-C-. In yet another embodiment of the present invention, X-Y is selected from the group consisting of-S-CH- and -O-CH-. In yet another embodiment of the present invention, X-Y is selected from the group consisting of S-CH-, -S-C(CH₃)-, -O-CH-, -O-C(CH₃)- and -N(CH₃)-CH-.

In an embodiment of the present invention, X- is -S-CH-. In another embodiment of the present invention X-Y is -CH=CH=CH-. In yet another embodiment of the present invention X-Y is -N(CH₃)-CH-. In yet another embodiment of the present invention X-Y is selected from the group consisting of -O-CH- and -O-C(CH₃)-.

In an embodiment, the present invention is directed to a compounds selected from the group consisting of *N*-(benzo[*b*]thien-3-ylmethyl)-sulfamide; *N*-[(5-chlorobenzo[*b*]thien-3-yl)methyl]-sulfamide; *N*-(3-benzofuranylmethyl)-sulfamide; *N*-[(5-fluorobenzo[b]thien-3-yl)methyl]-sulfamide; *N*-(1-benzo[*b*]thien-3-ylethyl)-sulfamide; *N*-(1-naphthalenylmethyl)-sulfamide; *N*-[(2-methyl-3-benzofuranyl)methyl]-sulfamide; *N*-[(5-bromobenzo[*b*]thien-3-yl)methyl]-sulfamide; *N*-[(4-bromobenzo[*b*]thien-3-yl)methyl]-sulfamide; *N*-[(7-fluorobenzo[*b*]thien-3-yl)methyl]-sulfamide; *N*-[(1-methyl-1*H*-indol-3-yl)methyl]-sulfamide; *N*-[(4-trifluoromethylbenzo[*b*]thien-3-yl)methyl]-sulfamide; *N*-[(4-cyanobenzo[b]thien-3-yl)methyl]-sulfamide; *N*-[(benzo[*b*]thien-3-yl)methyl]-sulfamoylpyrrolidine; *N*-[(benzo[*b*]thien-3-yl)methyl]-*N*'-ethylsulfamide; Imidazole-1-sulfonic acid [(benzo[b]thien-3-yl)methyl]-amide; and pharmaceutically acceptable salts thereof.

Additional embodiments of the present invention, include those wherein the substituents selected for one or more of the variables defined herein (i.e. R¹, R², R³, R⁴, X-Y and A) are independently selected to be any individual substituent or any subset of substituents selected from the complete list as defined herein.

In an embodiment of the present invention are compounds of formula (I) wherein the MES activity, measured according to the procedure described in Example 17, at 300 mg/kg dosing is greater than or equal to 3/5 mice at any time interval. In another embodiment of the present invention are compounds of formula (I) wherein the MES activity, measured according to the procedure described in Example 17, at 100 mg/kg dosing is greater than or equal to 3/5 mice at any time interval.

Representative compounds of the present invention are as listed in Table 1 and 2, below.

**Table 1: Representative Compounds of Formula (I)**

| | | | | | |
|---|---|---|---|---|---|
| 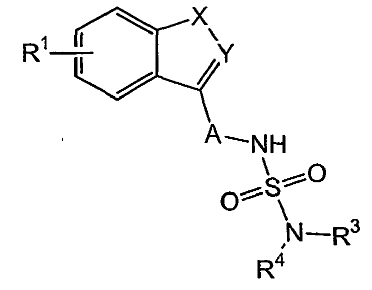 | | | | | |

| **ID No.** | **R¹** | **-X-Y-** | **A** | **R³** | **R⁴** |
|---|---|---|---|---|---|
| 1 | H | -S-CH- | -CH₂- | H | H |
| 3 | 5-Cl | -S-CH- | -CH₂- | H | H |
| 6 | H | -O-CH- | -CH₂- | H | H |
| 7 | H | -N(CH₃)-CH- | -CH₂- | H | H |
| 8 | 5-F | -S-CH- | -CH₂- | H | H |
| 9 | H | -S-CH- | -CH(CH₃)- | H | H |
| 10 | H | -CH=CH-CH- | -CH₂- | H | H |
| 13 | H | -O-C(CH₃) | -CH₂- | H | H |
| 15 | 5-Br | -S-CH- | -CH₂- | H | H |
| 17 | 4-Br | -S-CH- | -CH₂- | H | H |
| 18 | 7-F | -S-CH- | -CH₂- | H | H |
| 19 | 5-CF₃ | -S-CH- | -CH₂- | H | H |
| 20 | 5-CN | -S-CH- | -CH₂- | H | H |
| 21 | H | -S-CH- | -CH₂- | H | ethyl |

**Table 2**

| | | |
|---|---|---|
| 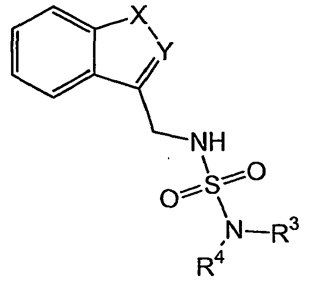 | | |

| **ID No.** | **-X-Y-** | **R3+R4 together with the N atom** |
|---|---|---|
| 101 | -S-CH- | *N*-pyrrolidinyl |
| 102 | -S-CH- | *N*-imidazolyl |

As used herein, "**halogen**" shall mean chlorine, bromine, fluorine and iodine.

As used herein, the term "**alkyl**" whether used alone or as part of a substituent group, include straight and branched chains. For example, alkyl radicals include methyl, ethyl, propyl, isopropyl, butyl, isobutyl, sec-butyl, t-butyl, pentyl and the like. Unless otherwise noted, **"C₁₋₄alkyl"** means a carbon chain composition of 1-4 carbon atoms.

When a particular group is "**substituted**" (e.g., alkyl, phenyl, aryl, heteroalkyl, heteroaryl), that group may have one or more substituents, preferably from one to five substituents, more preferably from one to three substituents, most preferably from one to two substituents, independently selected from the list of substituents.

With reference to substituents, the term "**independently**" means that when more than one of such substituents is possible, such substituents may be the same or different from each other.

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

As used herein, unless otherwise noted, the term **"leaving group"** shall mean a charged or uncharged atom or group which departs during a substitution or displacement reaction. Suitable examples include, but are not limited to, Br, CI, I, mesylate, tosylate, and the like.

Unless otherwise noted, the position at which the R¹ substituent is bound will be determined by counting around the core structure in a clockwise manner beginning at the X-Y positions as 1,2 and continuing from thereon as follows:

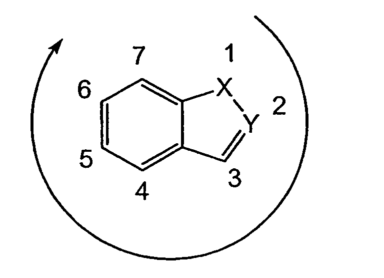

Should the X-Y substituent be -CH=CH-CH-, then the X-Y group will be counted as 1, 2, 3 and counting then continued clockwise around the core structure as previously noted.

Under standard nomenclature used throughout this disclosure, the terminal portion of the designated side chain is described first, followed by the adjacent functionality toward the point of attachment. Thus, for example, a "phenylC₁-C₆alkylaminocarbonylC₁-C₆alkyl" substituent refers to a group of the formula

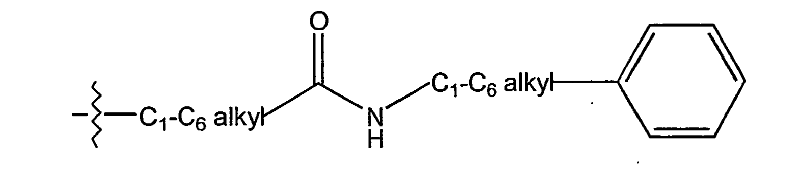

Abbreviations used in the specification, particularly the Schemes and Examples, are as follows:

| | |
|---|---|
| DCE | = Dichloroethane |
| DCM | = Dichloromethane |
| DMF | = N,N-Dimethylformamide |
| DMSO | = Dimethylsulfoxide |
| LAH | = Lithium Aluminum Hydride |
| MTBE | = Methyl-*tert*-butyl ether |
| THF | = Tetrahydrofuran |
| TLC | = Thin Layer Chromatography |

As used herein, unless otherwise noted, the terms "**epilepsy and related disorders"** or **"epilepsy or related disorder"** shall mean any disorder in which a subject (preferably a human adult, child or infant) experiences one or more seizures and / or tremors. Suitable examples include, but are not limited to, epilepsy (including, but not limited to, localization-related epilepsies, generalized epilepsies, epilepsies with both generalized and local seizures, and the like), seizures as a complication of a disease or condition (such as seizures associated with encephalopathy, phenylketonuria, juvenile Gaucher's disease, Lundborg's progressive myoclonic epilepsy, stroke, head trauma, stress, hormonal changes, drug use or withdrawal, alcohol use or withdrawal, sleep deprivation, and the like), essential tremor, restless limb syndrome, and the like. Preferably, the disorder is selected from epilepsy (regardless of type, underlying cause or origin), essential tremor or restless limb syndrome, more preferably, the disorder is epilepsy (regardless of type, underlying cause or origin) or essential tremor.

The term "**subject**" as used herein, refers to an animal, preferably a mammal, most preferably a human, who has been the object of treatment, observation or experiment.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.

Where the compounds according to this invention have at least one **chiral center**, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

Prodrugs will be functional derivatives of the compounds which are readily convertible *in vivo* into the required compound. Thus, in methods of treatment the term "administering" shall encompass the treatment of the various disorders described with the compound specifically disclosed or with a compound which may not be specifically disclosed, but which converts to the specified compound *in vivo* after administration to the patient Conventional procedures for the selection and preparation of suitable prodrug derivatives are described, for example, in "Design of Prodrugs", ed. H. Bundgaard, Elsevier, 1985.

For use in medicine, the salts of the compounds of this invention refer to non-toxic **"pharmaceutically acceptable salts."** Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutical acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts; Thus, representative pharmaceutically acceptable salts include the following:
acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:
acids including acetic acid, 2,2-dichlorolactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Compounds of formula (I) wherein A is -CH₂- may be prepared according to the process outlined in Scheme 1.

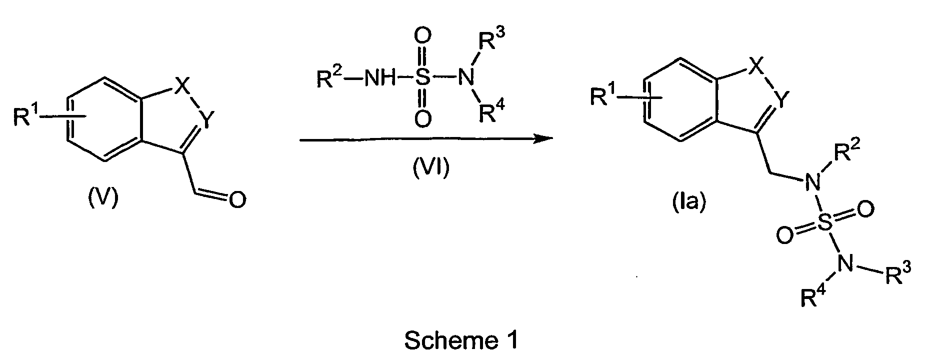

Accordingly, a suitably substituted compound of formula (V), a known compound or compound prepared by known methods, is reacted with a suitably substituted compound of formula (VI), a known compound or compound prepared by known methods, wherein the compound of formula (VI) is present in an amount in the range of about 2 to about 5 equivalents, in an organic solvent such as ethanol, methanol, dioxane, and the like, preferably, in an anhydrous organic solvent, preferably, at an elevated temperature in the range of about 50°C to about 100°C, more preferably at about reflux temperature, to yield the corresponding compound of formula (la).

Compounds of formula (I) may alternatively be prepared according to the process outlined in Scheme 2.

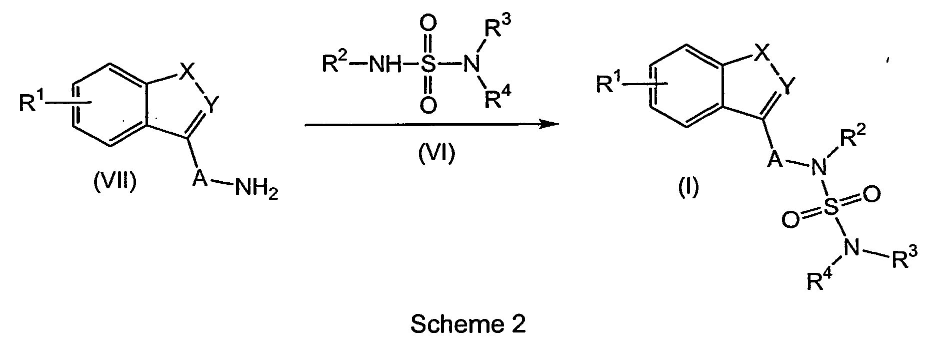

Accordingly, a suitably substituted compound of formula (VII), a known compound or compound prepared by known methods, is reacted with a suitably substituted compound of formula (VI), a known compound or compound prepared by known methods, wherein the compound of formula (VI) is present in an amount in the range of about 2 to about 5 equivalents, in an organic solvent such as THF, dioxane, and the like, preferably, in an anhydrous organic solvent, preferably, at an elevated temperature in the range of about 50°C to about 100°C, more preferably at about reflux temperature, to yield the corresponding compound of formula (I).

Compounds of formula (VII) wherein A is -CH₂- may, for example, be prepared by according to the process outlined in Scheme 3.

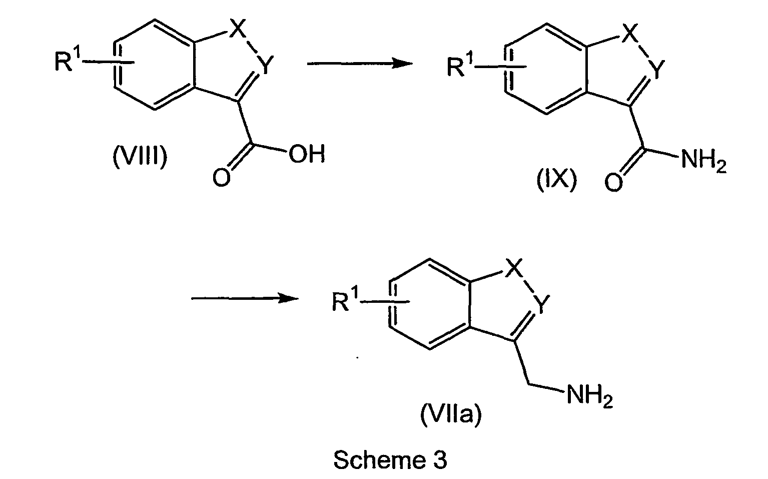

Accordingly, a suitably substituted a compound of formula (VIII), a known compound or compound prepared by known methods is reacted with an activating agent such as oxalyl chloride, sulfonyl chloride, and the like, and then reacted with an amine source such as ammonia, ammonium hydroxide, and the like, in an organic solvent such as THF, diethyl ether, DCM, DCE, and the like, to yield the corresponding compound of formula (IX).

The compound of formula (IX) is reacted with a suitably selected reducing agent such as LAH, borane, and the like, in an organic solvent such as THF, diethyl ether, and the like, to yield the corresponding compound of formula (VIIa).

Compounds of formula (VII) wherein A is -CH(CH₃)- may, for example, be prepared according to the process outlined in Scheme 4.

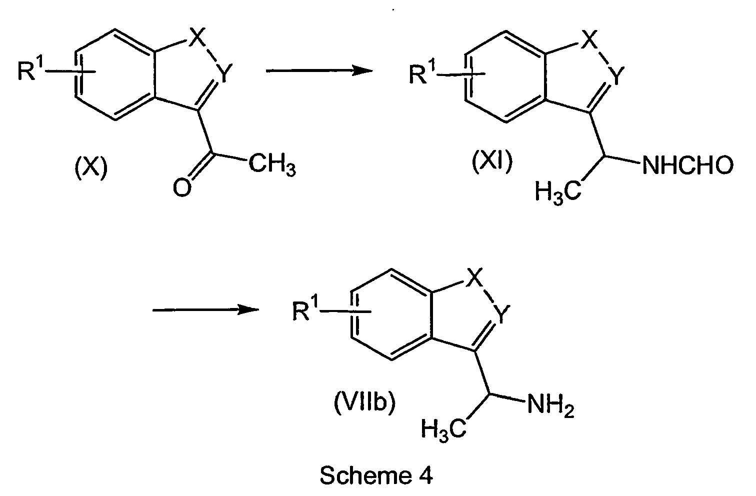

Accordingly, a suitably substituted compounds of formula (X), a known compound or compound prepared by known methods, is reacted with a mixture of formamide and formic acid, wherein the mixture of formamide and formic acid is present in an amount greater than about 1 equivalent, preferably, in an excess amount of greater than about 5 equivalent, at an elevated temperature of about 150°C, to yield the corresponding compound of formula (XI).

The compound of formula (XI) is hydrolyzed by reacting with concentrated HCl, concentrated H₂SO₄, and the like, at an elevated temperature, preferably at reflux temperature, to yield the corresponding compound of formula (VIIb).

Compounds of formula (VII) may alternatively, be prepared according to the process outlined in Scheme 5.

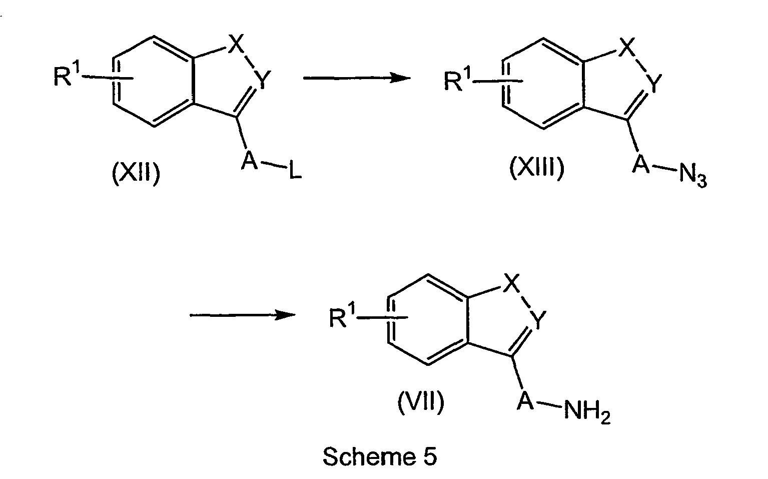

Accordingly, a suitably substituted compound of formula (XII), wherein L is a leaving group such as Br, Cl, I, tosylate, mesylate, and the like, a known compound or compound prepared by known methods, is reacted with sodium azide, in an organic solvent such a DMF, DMSO, methanol, ethanol, and the like, to yield the corresponding compound of formula (XIII).

The compound of formula (XIII) is reacted with a suitably selected reducing agent such as LAH, triphenylphosphine, H₂(g), and the like, according to known methods, to yield the corresponding compound of formula (VII).

Compounds of formula (VII) wherein A is CH₂ and X-Y is -O-CH₂- may, for example, be prepared according to the process outlined in Scheme 6.

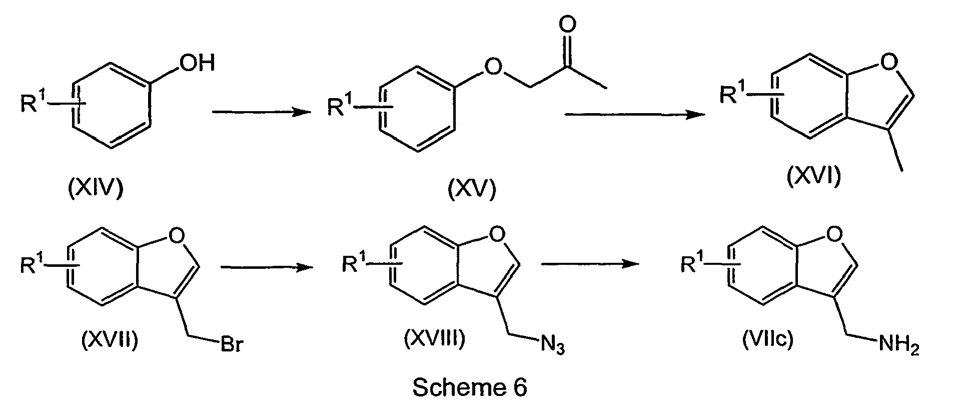

Accordingly, a suitably substituted phenol, a compound of formula (XIV), a known compound or compound prepared by known methods is reacted with bromoacetone, a known compound, in the presence of a base such as K₂CO₃, Na₂CO₃, NaH, triethylamine, pyridine, and the like, in an organic solvent such as acetonitrile, DMF, THF, and the like, optionally at an elevated temperature, to yield the corresponding compound of formula (XV).

The compound of formula (XV) is reacted with an acid such as polyphosphoric acid, sulfuric acid, hydrochloric acid, and the like, preferably with polyphosphoric acid, preferably in the absence of a solvent (one skilled in the art will recognize that the polyphosphoric acid acts as the solvent), to yield the corresponding compound of formula (XVI).

The compound of formula (XVI) is reacted with a source of bromine such as N-bromosuccinimide in the presence of benzoylperoixde, Br₂, and the like, in an organic solvent such as carbon tetrachloride, chloroform, DCM, and the like, preferably in a halogenated organic solvent, to yield the corresponding compound of formula (XVII).

The compound of formula (XVII) is reacted with sodium azide, in an organic solvent such a DMF, DMSO, methanol, ethanol, and the like, to yield the corresponding compound of formula (XVIII).

The compound of formula (XVIII) is reacted with a suitably selected reducing agent such as LAH, triphenylphosphine, H_{2(g)}, and the like, according to known methods, to yield the corresponding compound of formula (Vllc).

Compounds of formula (V) wherein X-Y is -S-CH- may, for example, be prepared according to the process outlined in Scheme 7.

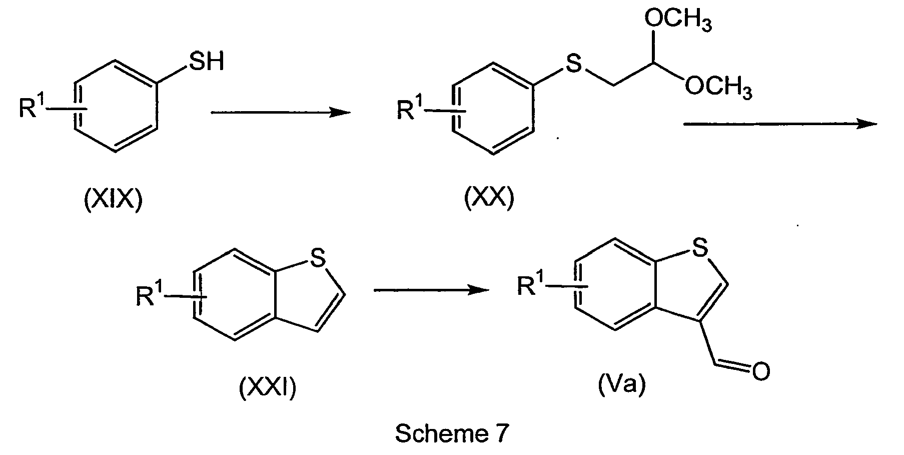

Accordingly, a suitably substituted compound of formula (XIX), a known compound or compound prepared by known methods is reacted with choroacetaldehyde dimethyl acetal or bromoacetaldehyde dimethyl acetal, a known compound, in the presence of a base such as potassium-tert-butoxide, sodium-tert-butxide, potassium carbonate, potassium hydroxide, and the like, in an organic solvent such as THF, DMF, acetonitrile, and the like, to yield the corresponding compound of formula (XX).

The compound of formula (XX) is reacted with reacted with an acid such as polyphosphoric acid, sulfuric acid, hydrochloric acid, and the like, preferably with polyphosphoric acid in the presence of chlorobenzene, preferably in the absence of a solvent (one skilled in the art will recognize that the polyphosphoric acid and / or the chlorobenzene may act as the solvent), at an elevated temperature in the range of from about 100 to 200°C, preferably at an elevated temperature of about reflux temperature, to yield the corresponding compound of formula (XXI).

The compound of formula (XXI) is reacted with a formylating reagent such as dichloromethyl methyl ether, and the like, in the presence of Lewis acid catalyst such as titanium tetrachloride, aluminum trichloride, tin tetrachloride, and the like, in an organic solvent such as DCM, chloroform, and the like, at a temperature in the range of from about 0°C to about room temperature, to yield the corresponding compound of formula (Va).

Compounds of formula (I) wherein R³ and / or R⁴ are other than hydrogen or R³ and R⁴ are taken together with the nitrogen to which they are bound to form a ring structure, may alternatively be prepared according to the process outlined in Scheme 8.

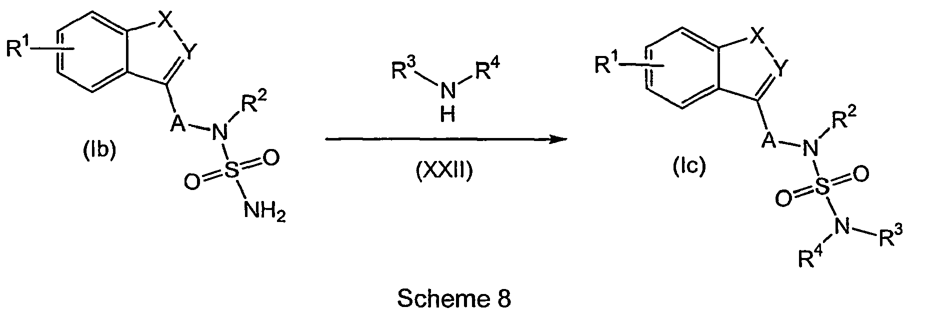

Accordingly, a suitably substituted compound of formula (Ib), is reacted with a suitably substituted amine, a compound of formula (XXII), a known compound or compound prepared by known methods, in water or an organic solvent such as dioxane, ethanol, THF, isopropanol, and the like, provide that the compound of formula (Ib) and the compound of formula (XXII) are at least partially soluble in the water or organic solvent, at a temperature in the range of from about room temperature to about reflux, preferably at about reflux temperature, to yield the corresponding compound of formula (lc).

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

The present invention further comprises pharmaceutical compositions containing one or more compounds of formula (I) with a pharmaceutically acceptable carrier. Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 50-100 mg and may be given at a dosage of from about 0.01-20.0 mg/kg/day, preferably from about 0.1 to 10 mg/kg/day, more preferably from about 0.5-5 mg/kg/day, more preferably from about 1.0-5.0 mg/kg/day. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

A method of treating epilepsy and related disorders described herein may also be carried out using a pharmaceutical composition comprising any of the compounds as defined herein and a pharmaceutically acceptable carrier. The pharmaceutical composition may contain between about 0.1 mg and 1000 mg, preferably about 50 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders: lubricants, disintegrating agents and coloring agents can also be incorporated into the mixture. Suitable binders Include. Without limitation, starch, gelatin, natural sugars such as glucose or beta- lactose, com sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

Compounds of this invention may be administered in any of the foregoing compositions and according to dosage regimens established in the art whenever treatment of epilepsy or related disorders is required.

The daily dosage of the products may be varied over a wide range from 0.01 to 1,000 mg per adult human per day. For oral administration, the compositions are preferably provided in the form of tablets containing, 0.01,0.05, 0.1, 0.5, 1.0, 2.5, 5.0, 10.0, 15.0, 25.0, 50.0, 100, 150, 200, 250, 500 and 1000 milligrams of the active ingredient for the symptomatic adjustment of the dosage to the patient to be treated. An effective amount of the drug is ordinarily supplied at a dosage level of from about 0.01 mg/kg to about 20 mg/kg of body weight per day. Preferably, the range is from about 0.5 to about 10.0 mg/kg of body weight per day, most preferably, from about 1.0 to about 5.0 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound to treat or prevent a given disorder.

One skilled in the art will further recognize that human clinical trails including first-in-human, dose ranging and efficacy trials, in healthy patients and / or those suffering from a given disorder, may be completed according to methods well known in the clinical and medical arts.

The following Examples are set forth to aid in the understanding of the invention, and are not intended and should not be construed to limit in any way the invention set forth in the claims which follow thereafter.

### Example 1

### N-(benzo[b]thien-3-ylmethyl)-sulfamide (Compound #1)

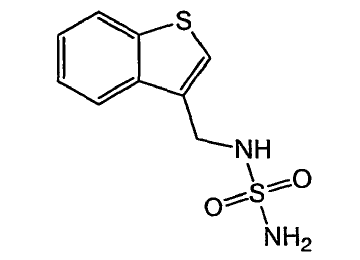

Thianaphthene-3-carboxaldehyde (1.62 g, 10.0 mmol) was dissolved in anhydrous ethanol (50 mL). Sulfamide (4.0 g, 42 mmol) was added and the mixture was heated to reflux for 16 hours. The mixture was cooled to room temperature. Sodium borohydride (0.416 g, 11.0 mmol) was added and the mixture was stirred at room temperature for three hours. The reaction was diluted with water (50 mL) and extracted with chloroform (3 x 75 mL). The extracts were concentrated and chromatographed (5% methanol in DCM) to yield the title compound as a white solid.

¹H NMR (DMSO-*d*₆): δ 7.98 (1 H, dd, *J* = 6.5, 2.3 Hz), 7.92 (1 H, dd, *J* = 6.6, 2.4 Hz), 7.62 (1 H, s), 7.36-7.45 (2H, m), 7.08 (1 H, t, J = 6.3 Hz), 6.72 (2H, s), 4.31 (2H, d, J = 6.3 Hz).

### Example 2

### N-[(5-chlorobenzo[b]thien-3-yl)methyl]-sulfamide (Compound #3)

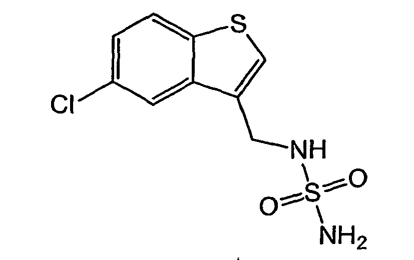

(5-Chloro-1-benzothiophene-3-yl)methylamine (0.820 g, 4.15 mmol) and sulfamide (2.5 g, 26 mmol) were combined in anhydrous dioxane (50 mL) and the mixture was heated to reflux for four hours. The reaction was cooled and diluted with water (50 mL). The solution was extracted with chloroform (3 x 75 mL). The extracts were concentrated and chromatographed (5% methanol in DCM) to yield the title compound as a white solid.

¹H NMR (DMSO-*d*₆): δ 8.05 (2H, m), 7.74 (1 H, s), 7.40 (1 H, d, J = 6.5 Hz), 7.07 (1 H, t, J = 6.3 Hz), 6.72 (2H, s), 4.26 (2H, d, J = 6.4 Hz).

### Example 3

### N-[(1-methyl-1H-indol-3-yl)methyl]-sulfamide (Compound #7)

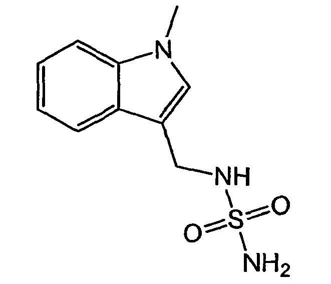

N-Methylindole-3-carboxaldehyde (1.66 g, 10.4 mmol) was dissolved in anhydrous ethanol (50 mL). Sulfamide (4.5 g, 47 mmol) was added and the mixture was heated to reflux for 16 hours. Additional sulfamide (1.0 g, 10.4 mmol) was added and the mixture was heated to reflux for 24 hours. The mixture was cooled to room temperature. Sodium borohydride (0.722 g, 12.5 mmol) was added and the mixture was stirred at room temperature for one hour. The reaction was diluted with water (50 mL) and extracted with DCM (3 x 75 mL). The extracts were concentrated and about 1 mL of methanol was added to create a slurry which was filtered to yield the title compound as a white powder.

¹H NMR (CD₃OD): δ 7.67 (1H, d, J = 5.9 Hz), 7.32 (1 H, d, J = 6.2 Hz), 7.14-7.19 (2H, m), 7.06 (1 H, dt, J = 7.7, 0.7 Hz), 4.36 (2H, s), 3.75 (3H, s) MS (M-H)⁻ 237.6.

### Example 4

### N-(3-benzofuranylmethyl)-sulfamide (Compound #6)

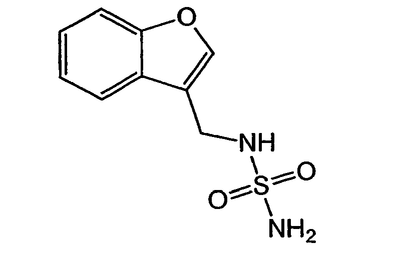

Benzofuran-3-carboxylic acid (1.91 g, 11.8 mmol) was suspended in anhydrous DCM (75 mL). Oxalyl chloride (2.0 M in DCM, 6.48 mL) and then one drop of dimethylformamide were added. The solution was stirred at room temperature for two hours, then ammonium hydroxide (concentrated, 10 mL) was added. The resulting mixture was diluted with water (100 mL) and extracted with DCM (3 x 100 mL). The extracts were concentrated to a gray solid and dissolved in anhydrous THF (100 mL). Lithium aluminum hydride (1.0 M in THF, 11.8 mL) was added. The mixture was stirred at room temperature for 16 hours. A minimal amount of saturated aqueous NaHCO₃ and then MgSO₄ were added. The mixture was filtered and then extracted with 1 N HCl. The aqueous extracts were adjusted to pH 14 with 3N NaOH and extracted with DCM. The organic extracts were dried with magnesium sulfate and concentrated to a colorless oil. The oil was dissolved in dioxane (50 mL) and sulfamide (3.7 g, 38 mmol) was added. The mixture was heated to reflux for 4 hours, cooled to room temperature, and concentrated. The resulting solid was chromatographed (5% methanol in DCM) to yield the title compound as a slightly yellow solid.

¹H NMR (CD₃OD): δ 7.53 (1 H, d, *J* = 5.7 Hz), 7.44 (1 H, d, *J* = 6.0 Hz), 7.16-7.26 (2H, m), 6.73 (1H, s), 4.35 (2H, s).

### Example 5

### N-[(5-fluorobenzo[b]thien-3-yl)methyl]-sulfamide (Compound #8)

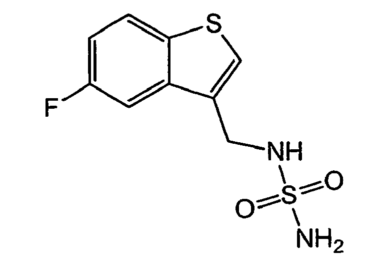

5-Fluoro-3-methylbenzothiophene (1.14 g, 6.83 mmol), benzoyl peroxide (0.165 g, 0.68 mmol) and N-bromosuccinimide (1.70 g, 7.52 mmol) were combined in carbon tetrachloride (25 mL) and the mixture was heated to reflux for 3 hours. The yellow solution was cooled, diluted with water, and extracted with DCM (2 x 50 mL). The extracts were washed with brine (100 mL), dried with magnesium sulfate, and concentrated to an orange solid. The solid was dissolved in anhydrous DMF. Sodium azide (4.0 g, 61 mmol) was added and the mixture was stirred for 16 hours at room temperature. The reaction was diluted with water (100 mL) and extracted with diethyl ether (2 x 75 mL). The extracts were washed with brine (100 mL), dried with magnesium sulfate, and concentrated to a yellow oil. The oil was dissolved in a mixture of THF (50 mL) and water (5 mL). Triphenylphosphine (3.60 g, 13.7 mmol) was added. The mixture was stirred at room temperature for 16 hours. The reaction was concentrated and chromatographed (2 to 5% methanol in DCM). The resulting *C*-(5-fluoro-benzo[b]thien-3-yl)-methylamine (1.04 g, 5.73 mmol) was dissolved in anhydrous dioxane (50 mL) and sulfamide (2.75 g, 28.7 mmol) was added. The reaction was heated to reflux for 4 hours, cooled to room temperature, and concentrated to a solid which was chromatographed (5% methanol in DCM) to yield the title compound as a white solid.

¹H NMR (CD₃OD): δ 7.85 (1 H, dd, J = 6.6, 3.6 Hz), 7.66 (1 H, dd, J = 7.4, 1.8 Hz), 7.62 (1 H, s), 7.13-7.18 (1 H, m), 4.40 (2H, s).

### Example 6

### N-(1-benzo[b]thien-3-ylethyl)-sulfamide (Compound #9)

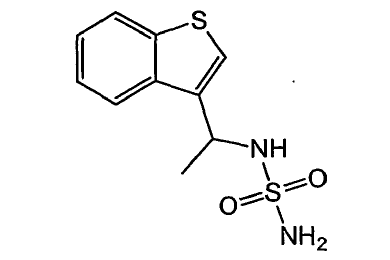

3-Acetylthianaphthene (3.00 g, 17.0 mmol) was added to a mixture of formic acid (10 mL) and formamide (10 mL). The solution was heated to 150°C for 8 hours. The reaction was cooled to room temperature, diluted with water (50 mL), and extracted with diethyl ether (3 x 50 mL). The ether extracts were washed with saturated aqueous NaHCO₃ and brine. The solution was concentrated and chromatographed (5% methanol in DCM) to yield *N*-(1-benzo[b]thiophen-3-yl-ethyl)-formamide (1.76 g) as a white solid which was suspended in concentrated HCl (30 mL). The mixture was heated to reflux for 1.5 hours then diluted with water (100 mL). 3N NaOH was added until the pH was 14. The mixture was extracted with diethyl ether (3 x 100 mL) then dried with magnesium sulfate and concentrated to an orange oil. The oil was dissolved in anhydrous dioxane (75 mL) and sulfamide was added. The mixture was heated to reflux for 2 hours then diluted with water (50 ml). The solution was extracted with ethyl acetate (2 x 50 mL), dried with magnesium sulfate, concentrated, and chromatographed (2.5% to 5% methanol in DCM) to yield the title compound as a white solid.

¹H NMR (CD₃OD): δ 8.01 (1 H, dd, J = 5.5, 0.7 Hz), 7.85 (1 H, dt, J = 6.0, 0.6 Hz), 7.49 (1 H, s), 7.31-7.40 (2H, m), 4.95 (1 H, q, J = 5.1 Hz), 1.67 (3H, d, J = 5.1 Hz).

### Example 7

### N-(1-naphthalenylmethyl)-sulfamide (Compound #10)

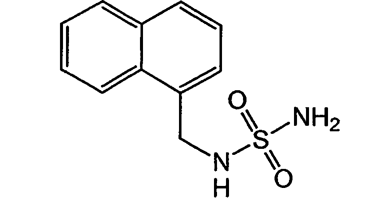

1-Naphthanlenemethylamine (2.00 g, 12.7 mmol) and sulfamide (5.0 g, 52 mmol) were combined in anhydrous dioxane (100 mL) and the mixture was heated to reflux for 6 hours. The reaction was cooled to room temperature and was filtered. The filtrate was concentrated to a solid and washed with water until TLC indicated no remaining trace of sulfamide in the solid. The collected solid was dried under vacuum to yield the title compound as a white solid.

¹H NMR (CDCl₃): δ 8.09 (1 H, d, J = 6.3 Hz), 7.86 (1 H, dd, J = 12.9, 6.2 Hz), 7.42-7.61 (4H, m), 4.75 (2H, d, J = 4.4 Hz), 4.58 (1 H, br s), 4.51 (2H, br s).

### Example 8

### N-[(2-methyl-3-benzofuranyl)methyl]-sulfamide (Compound #13)

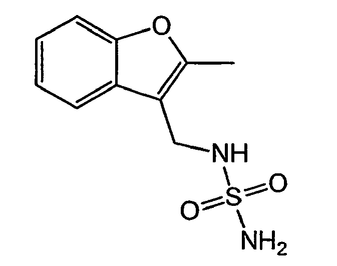

2-Methylbenzofuran-3-carbaldehyde (0.51 g, 3.18 mmol) was dissolved in anhydrous ethanol (25 mL). Sulfamide (1.5 g, 16 mmol) was added and the mixture was heated to reflux for 4 days. The mixture was cooled to room temperature. Sodium borohydride (0.132 g, 3.50 mmol) was added and the mixture was stirred at room temperature for 24 hours. The reaction was diluted with water (100 mL) and extracted with DCM (3 x 75 mL). The extracts were concentrated and suspended in a minimal amount of DCM and filtered to yield the title compound as a white solid.

¹H NMR (DMSO-*d*₆): δ 7.65 (1 H, dd, *J* = 6.4, 2.6 Hz), 7.43-7.47 (1 H, m), 7.19-7.23 (2H, m), 6.87 (1 H, t, J = 6.2 Hz), 6.68 (2H, s), 4.11 (2H, d, J = 6.2 Hz), 2.42 (3H, s).

### Example 9

### N-[(5-bromobenzo[b]thien-3-yl)methyl]-sulfamide (Compound #15)

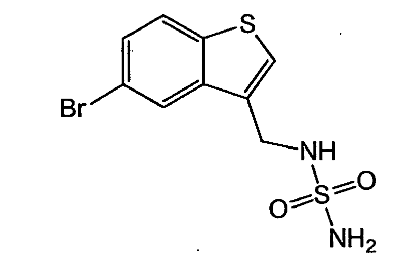

5-Bromobenzothiophene (1.60 g, 7.51 mmol) and dichloromethyl methyl ether (1.29 g, 11.3 mmol) were dissolved in anhydrous 1,2-dichloroethane (75 mL). Titanium tetrachloride (2.14 g, 11.3 mmol) was added, turning the solution dark. After one hour at room temperature, the reaction was poured into a mixture of saturated aqueous NaHCO₃ and ice. The mixture was stirred for about 30 minutes and then was extracted with DCM (2 x 100 mL). The extracts were concentrated and chromatographed (0 to 5% ethyl acetate in hexane) to yield 5-bromo-benzo[b]thiophene-3-carbaldehyde (1.32 g). The 5-bromobenzothiophene-3-carboxaldehyde (1.20 g, 4.98 mmol) and sulfamide (4.0 g, 42 mmol) were combined in anhydrous ethanol (25 mL) and heated to reflux for three days. The reaction was cooled to room temperature and sodium borohydride (0.207 g, 5.47 mmol) was added. After five hours, water (50 ml) was added and the solution was extracted with chloroform (3 x 50 mL). The extracts were concentrated, suspended in a minimal amount of DCM, and filtered to provide the title compound as a yellow solid.

¹H NMR (DMSO-*d*₆): δ 8.12 (1H, d, *J* = 1.8 Hz), 7.97 (1H, d, *J* = 8.6), 7.71 (1H, s), 7.52 (1H, dd, *J* = 8.6, 1.9 Hz), 7.12 (1H, t, *J* = 6.3 Hz), 6.72 (2H, s), 4.28 (2H, d, J = 6.2 Hz).

### Example 10

### N-[(4-bromobenzo[b]thien-3-yl)methyl]-sulfamide (Compound #17)

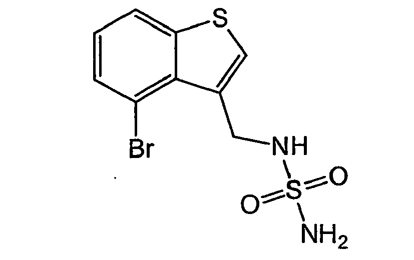

4-Bromobenzothiophene (1.80 g, 8.45 mmol) and dichloromethyl methyl ether (1.46 g, 12.7 mmol) were dissolved in anhydrous DCM (100 mL). Titanium tetrachloride (2.40 g, 12.7 mmol) was added, turning the solution dark. After 30 minutes at room temperature, the reaction was poured into a mixture of saturated aqueous NaHCO₃ and ice. The mixture was stirred for about 30 minutes and then was extracted with DCM (2 x 150 mL). The extracts were concentrated and chromatographed (0 to 15% ethyl acetate in hexane) to yield 4-bromobenzothiophene-3-carboxaldehyde (0.910 g). The 4-bromobenzothiophene-3-carboxaldehyde (0.910 g, 3.77 mmol) and sulfamide (3.0 g, 31 mmol) were combined in anhydrous ethanol (25 mL) and heated to reflux for three days. The reaction was cooled to room temperature and sodium borohydride (0.157 g, 4.15 mmol) was added. After five hours, water (50 ml) was added and the solution was extracted with chloroform (3 x 50 mL). The extracts were concentrated, suspended in a minimal amount of DCM, and filtered to yield the title compound as a yellow solid.

¹H NMR (DMSO-*d*₆): δ 8.05 (1H, dd, *J* = 8.1, 0.8 Hz), 7.78 (1H, s), 7.64 (1H, dd, *J* = 7.6, 0.8 Hz), 7.27 (1H, t, *J* = 7.9 Hz), 7.13 (1H, t, *J* = 6.3 Hz), 6.72 (2H, br s), 4.65 (2H, d, J = 5.3 Hz).

### Example 11

### N-[(7-fluorobenzo[b]thien-3-yl)methyl]-sulfamide (Compound #18)

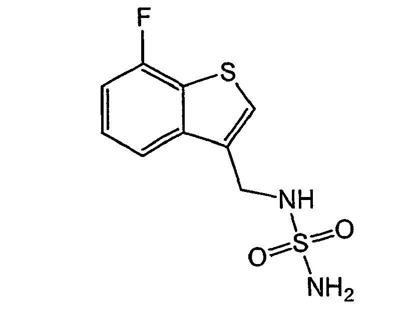

2-Fluorothiophenol (4.14 g, 32.6 mmol) was dissolved in anhydrous THF (100 mL). Potassium tert-butoxide (1.0 M in THF, 35.8 mL) was added and the suspension was stirred at room temperature for 15 minutes. 2-Chloroacetaldehyde dimethyl acetal was added and the mixture was stirred for 3 days. Water (100 mL) was added and the solution was extracted with diethyl ether (3 x 100 mL). The extracts were concentrated to a yellow oil and chromatographed (5 to 20% ethyl acetate in hexane) to yield 1-(2,2-dimethoxyethylsulfanyl)-2-fluoro-benzene (6.42 g) as a colorless oil. Chlorobenzene (25 mL) was heated to reflux and polyphosphoric acid (1 mL) was added. The 1-(2,2-dimethoxy-ethylsulfanyl)-2-fluoro-benzene was then added slowly turning the solution dark. After 3 hours of heating, the reaction was cooled to room temperature and diluted with water (50 mL). The solution was extracted with benzene (2 x 50 mL). The extracts were concentrated and chromatographed (0 to 15% ethyl acetate in hexane) to yield 7-fluorobenzothiophene (0.77 g). The 7-fluorobenzothiophene (0.77 g, 5.1 mmol) and dichloromethyl methyl ether (0.872 g, 7.6 mmol) were dissolved in anhydrous DCM (25 mL). Titanium tetrachloride (1.0 M in DCM, 7.6 mL, 7.6 mmol) was added, turning the solution dark. After 30 minutes at room temperature, the reaction was poured into a mixture of saturated aqueous NaHCO₃ and ice. The mixture was stirred for about 30 minutes and then was extracted with DCM (2 x 50 mL). The extracts were concentrated and chromatographed (0 to 15% ethyl acetate in hexane) to yield 7-fluorobenzothiophene-3-carboxaldehyde (0.642 g). The 7-fluorobenzothiophene-3-carboxaldehyde (0.642 g, 3.77 mmol) and sulfamide (1.7 g, 18 mmol) were combined in anhydrous ethanol (20 mL) and heated to reflux for three days. The reaction was cooled to room temperature and sodium borohydride (0.148 g, 3.92 mmol) was added. After two hours, water (25 ml) was added and the solution was extracted with chloroform (3 x 25 mL). The extracts were concentrated, suspended in a minimal amount of DCM, and filtered to yield the title compound as a yellow solid.

¹H NMR (DMSO-*d*₆): δ 7.78 (1H, d, *J* = 8.0 Hz), 7.43-7.50 (1H, m), 7.27 (1 H, dd, J = 10.3, 7.9 Hz), 7.14 (1 H, t, J = 6.4 Hz), 6.74 (2H, br s), 4.31 (2H, d, J = 6.4 Hz).

### Example 12

### N-[(4-trifluoromethylbenzo[b]thien-3-yl)methyl]-sulfamide (Compound #19)

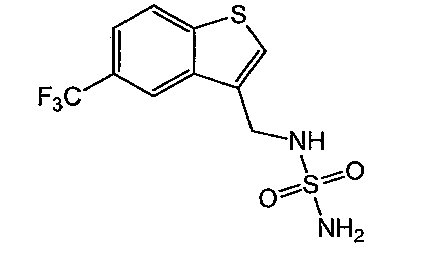

4-Trifluoromethylbenzothiophene (0.276 g, 1.37 mmol) and dichloromethyl methyl ether (0.236 g, 2.06 mmol) were dissolved in anhydrous DCM (10 mL). Titanium tetrachloride (1.0M in DCM, 2.1 mL, 2.1 mmol) was added, turning the solution dark. After 30 minutes at room temperature, the reaction was poured into a mixture of saturated aqueous NaHCO₃ and ice. The mixture was stirred for about 30 minutes and then extracted with DCM (2 x 25 mL). The extracts were concentrated and chromatographed (0 to 15% ethyl acetate in hexane) to yield 4-trifluoromethylbenzothiophene-3-carboxaldehyde.

The 4-trifluoromethylbenzothiophene-3-carboxaldehyde (0.226 g, 0.982 mmol) and sulfamide (0.471 g, 4.91 mmol) were combined in anhydrous ethanol (5 mL) and heated to reflux for 24 hours. The reaction was cooled to room temperature and sodium borohydride (0.056 g, 1.47 mmol) was added. After five hours, water (10 ml) was added and the solution was extracted with chloroform (3 x 10 mL). The extracts were concentrated, and chromatographed (5% methanol in DCM) to yield the title compound as a white solid.

¹H NMR (DMSO-*d*₆): δ 8.30 (1 H, s), 8.25 (1 H, d, J = 8.4 Hz), 7.84 (1 H, s), 7.68 (1 H, dd, J = 8.5, 1.4 Hz), 6.7-6.9 (2H, br s), 4.4-4.5 (1 H, br s), 4.37 (2H, s).

### Example 13

### N-[(4-cyanobenzo[b]thien-3-yl)methyl]-sulfamide (Compound #20)

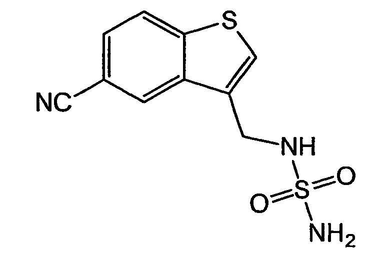

4-Cyanobenzothiophene (1.15 g, 7.22 mmol) and dichloromethyl methyl ether (1.25 g, 10.8 mmol) were dissolved in anhydrous DCM (100 mL). Titanium tetrachloride (1.0M in DCM, 10.8 mL, 10.8 mmol) was added, turning the solution dark. After 30 minutes at room temperature, the reaction was poured into a mixture of saturated aqueous NaHCO₃ and ice. The mixture was stirred for about 30 minutes and then was extracted with DCM (2 x 50 mL). The extracts were concentrated and chromatographed (0 to 15% ethyl acetate in hexane) to yield 4-cyanobenzothiophene-3-carboxaldehyde.

The 4-cyanobenzothiophene-3-carboxaldehyde (0.298 g, 1.59 mmol) and sulfamide (0.766 g, 7.97 mmol) were combined in anhydrous ethanol (20 mL) and heated to reflux for 24 hours. The reaction was cooled to room temperature and sodium borohydride (0.091 g, 2.39 mmol) was added. After five hours, water (20 ml) was added and the solution was extracted with chloroform (3 x 20 mL). The extracts were concentrated, and chromatographed (5% methanol in DCM) to yield the title compound as a white solid.

¹H NMR (DMSO-*d*₆): δ 8.37 (1 H, s), 8.30 (1 H, d, J = 8.4 Hz), 7.87 (1 H, s), 7.70 (1 H, dd, J = 8.5,1.4 Hz), 6.7-6.9 (2H, br s), 4.4-4.5 (1 H, br s), 4.40 (2H, s).

### Example 14

### N-[(benzo[b]thien-3-yl)methyl]-sulfamoylpyrrolidine (Compound #101)

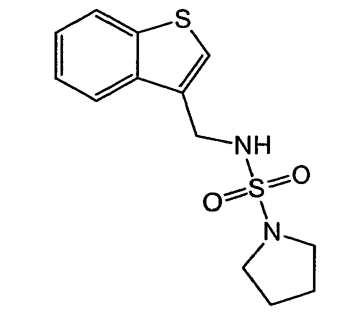

*N*-[(Benzo[*b*]thien-3-yl)methyl]-sulfamide (0.250 g, 1.03 mmol) and pyrrolidine (0.25 mL) were combined in anhydrous dioxane (5 mL) and heated to reflux for 32 hours. The reaction was evaporated and chromatographed with 5% methanol in DCM to yield the title compound as a white solid.

¹H NMR (CDCl₃): δ 7.84-7.89 (2H, m), 7.38-7.45 (3H, m), 4.49 (3H, br s), 3.25 (4H, t, J = 4.0 Hz), 1.80 (4H, t, J = 4.0 Hz).

### Example 15

### N-[(benzo[b]thien-3-yl)methyl]-N'-ethylsulfamide (Compound #21)

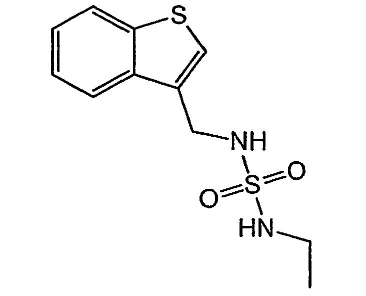

*N*-[(Benzo[*b*]thien-3-yl)methyl]-sulfamide (0.250 g, 1.03 mmol) and ethylamine (70% in H₂O, 0.10 mL) were combined in anhydrous dioxane (5 mL) and heated to reflux for 32 hours. The reaction was evaporated and chromatographed with 5% methanol in DCM to yield the title compound as a white solid.

¹H NMR (CDCl₃): δ 7.83-7.90 (2H, m), 7.36-7.47 (3H, m), 4.51 (2H, s), 2.90 (2H, q, J = 7 Hz), 1.03 (3H, t, J = 7 Hz).

### Example 16

### Imidazole-1-sulfonic acid [(benzo[b]thien-3-yl)methyl]-amide (Compound #102)

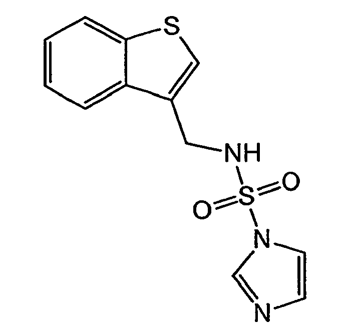

3-Benzothienylmethylamine and 3-(imidzole-1-sulfonyl)-1-methyl-3H-imidazol-1-ium triflate were combined in anhydrous acetonitrile. The solution was stirred at room temperature overnight, concentrated, and chromatographed (5% methanol in DCM) to yield the title compound as a tan solid.

¹H NMR (DMSO-d₆): δ 8.05 (1 H, dd, J = 7.0, 1.6 Hz), 7.99 (1 H, dd, J = 7.1, 1.7 Hz), 7.85 (1 H, s), 7.66 (1 H, s), 7.42-7.65 (5H, m), 4.34 (2H, s).

### Example 17

### In Vivo Assay: Maximal Electroshock Test (MES)

Anticonvulsant activity was determined using the MES test, run according to the procedure described in detail below. Swinyard EA, Woodhead JH, White HS, Franklin MR. Experimental selection, quantification, and evaluation of anticonvulsants. In Levy RH, et al., eds. Antiepileptic Drugs. 3rd ed. New York: Raven Press, 1989:85-102

CF-1 male albino mice (25-35g) were fasted for 16 hours before testing. Mice were randomly selected into control and test groups, with the animals dosed with vehicle or test compound, at varying concentrations, respectively. On the study date, at 30 minutes prior to shock, the mice were orally dosed with vehicle (0.5% methylcellulose) or test compound (100-300 mg/kg). Seizures were induced by trans-corneal electric shock using a 60-Hz alternating current, 50 mA, delivered for 0.2 sec. The mice in the test groups were subjected to electrical stimulus at time intervals between 15 minutes and 4 hours following administration of test compound. The shock resulted in an immediate full body tonic extension. The test was complete when the entire course of the convulsion has been observed (typically, less than 1 minute after electrical stimulation), and the mice were then immediately euthanized by carbon dioxide inhalation.

Abolition of the full body tonic extensor component of the seizure was taken as the endpoint of the test. Absence of this component indicated that the test compound had the ability to prevent the spread of seizure discharge through neural tissue. The ED₅₀ value of the test compound (calculated when appropriate) was the calculated dose required to block the hind limb tonic-extensor component of the MES-induced seizure in 50% of the rodents tested. A probit analysis was used to calculate the ED₅₀ and 95% fiducial limits (FL).

Representative compounds of the present invention were tested according to the procedure described above, with results as listed in Table 3 below. Results are listed as (number of mice with full body tonic extension prevented) / (total number of mice tested) (@ a given time).

**Table 3: MES Activity**

| **ID No.** | **MES @100 mpk** | **MES @ 300 mpk** |
|---|---|---|
| 1 | 1/5 (0.5h) | |
| | 3/5 (2h) | |
| | 2/5 (4h) | |
| 3 | inactive | 0/5 (0.5 hr) |
| | | 3/5 (2 hr) |
| | | 1/5 (4 hr) |
| 6 | inactive | 5/5 (0.5 hr) |
| | | 0/5 (2 hr) |
| | | 0/5 (4 hr) |
| 7 | inactive | 5/5 (0.5 hr) |
| | | 0/5 (2 hr) |
| | | 0/5 (4 hr) |
| 8 | 0/5 (0.5h) | |
| | 4/5 (2h) | |
| | 1/5 (4h) | |
| 9 | 1/5 (0.5h) | |
| | 0/5 (2h) | |
| | 0/5 (4h) | |
| 10 | 2/5 (0.5h) | |
| | 1/5 (2h) | |
| | 0/5 (4h) | |
| 13 | 4/5 (0.5h) | |
| | 0/5 (2h) | |
| | 0/5 (4h) | |
| 15 | | 0/3 (0.5 hr) |
| | | 0/3 (2 hr) |
| | | 1/3 (4 hr) |
| 17 | | 0/3 (0.5 hr) |
| | | 0/3 (2 hr) |
| | | 1/3 (4 hr) |
| 18 | | 0/3 (0.5 hr) |
| | | 2/3 (2 hr) |
| | | 0/3 (4 hr) |

### Example 18

As a specific embodiment of an oral composition, 100 mg of the Compound #1 prepared as in Example 1 is formulated with sufficient finely divided lactose to provide a total amount of 580 to 590 mg to fill a size O hard gel capsule.

While the foregoing specification teaches the principles of the present invention, with examples provided for the purpose of illustration, it Will be understood that the practice of the invention encompasses all of the usual variations, adaptations and/or modifications as come within the scope of the following claims.

## Claims

1. A compound of the formula (I) 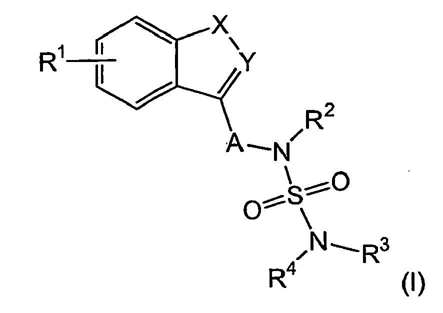 wherein
R¹ is selected from the group consisting of hydrogen, halogen, hydroxy, methoxy, trifluoromethyl, nitro and cyano;
X-Y is selected from the group consisting of -S-CH-, -S-C(CH₃)-, -O-CH-, -O-C(CH₃)-, -N(CH₃)-CH- and -CH=CH-CH-;
A is selected from the group consisting of -CH₂- and -CH(CH₃)-;
R² is selected from the group consisting of hydrogen and methyl;
R³ and R⁴ are each independently selected from the group consisting of hydrogen and C₁₋₄alkyl;
alternatively, R³ and R⁴ are taken together with the nitrogen atom to which they are bound to form a 5 to 7 membered, saturated, partially unsaturated or aromatic ring structure, optionally containing one to two additional heteroatoms independently selected from the group consisting of O, N and S;
or a pharmaceutically acceptable salt thereof.

2. A compound as in Claim 1, wherein
R¹ is selected from the group consisting of hydrogen, halogen, trifluoromethyl, cyano and nitro;
X-Y is selected from the group consisting of -S-CH-, -O-CH-, -O-C(CH₃)-, -N(CH₃)-CH- and -CH=CH-CH-;
A is selected from the group consisting of -CH₂- and -CH(CH₃)-;
R² is selected from the group consisting of hydrogen and methyl;
R³ and R⁴ are each independently selected from the group consisting of hydrogen, methyl and ethyl;
or a pharmaceutically acceptable salt thereof.

3. A compound as in Claim 2, wherein
R¹ is selected from the group consisting of hydrogen, halogen, trifluoromethyl and cyano;
X-Y is selected from the group consisting of-S-CH-, -O-CH-, -O-C(CH₃)-, -N(CH₃)-CH- and -CH=CH-CH-;
A is selected from the group consisting of -CH₂- and -CH(CH₃)-;
R² is hydrogen;
R³ and R⁴ are each independently selected from the group consisting of hydrogen and ethyl;
or a pharmaceutically acceptable salt thereof.

4. A compound as in Claim 3, wherein
R¹ is selected from the group consisting of hydrogen, 5-chloro, 5-fluoro, 5-bromo, 4-bromo, 7-fluoro, 5-trifluoromethyl and 5-cyano;
X-Y is selected from the group consisting of-S-CH-, -O-CH-, -O-C(CH₃)-, -N(CH₃)-CH- and -CH=CH-CH-;
A is selected from the group consisting of -CH₂- and -CH(CH₃)-;
R² is hydrogen;
R³ and R⁴ are each hydrogen; alternatively R³ is hydrogen and R⁴ is ethyl;
or a pharmaceutically acceptable salt thereof.

5. A compound as in Claim 1, wherein
R¹ is selected from the group consisting of hydrogen, halogen, trifluoromethyl and cyano;
X-Y is selected from the group consisting of -S-CH-, -O-CH-, -O-C(CH₃)-, -N(CH₃)-CH- and -CH=CH-CH-;
A is selected from the group consisting of -CH₂- and -CH(CH₃)-;
R² is selected from the group consisting of hydrogen and methyl;
R³ and R⁴ are taken together with the nitrogen atom to which they are bound to form a 5 to 7 membered, saturated, partially unsaturated or aromatic ring structure, optionally containing one to two additional heteroatoms independently selected from the group consisting of O, N and S;
or a pharmaceutically acceptable salt thereof.

6. A compound as in Claim 5, wherein
R¹ is selected from the group consisting of hydrogen, halogen, trifluoromethyl and cyano;
X-Y is selected from the group consisting of-S-CH-, -O-CH-, -O-C(CH₃)-, -N(CH₃)-CH- and -CH=CH-CH-;
A is selected from the group consisting of -CH₂- and -CH(CH₃)-;
R² is selected from the group consisting of hydrogen and methyl;
R³ and R⁴ are taken together with the nitrogen atom to which they are bound to form a 5 to 6 membered, saturated or aromatic ring structure, optionally containing one to two additional heteroatoms independently selected from the group consisting of O, N and S;
or a pharmaceutically acceptable salt thereof.

7. A compound as in Claim 6, wherein
R¹ is hydrogen;
X-Y is -S-CH-;
A is-CH₂-;
R² is hydrogen;
R³ and R⁴ are taken together with the nitrogen atom to which they are bound to form a 5 membered ring structure selected from the group consisting of pyrrolidinyl and imidazolyl;
or a pharmaceutically acceptable salt thereof.

8. A compound as in Claim 2, selected from the group consisting of
*N*-(benzo[*b*]thien-3-ylmethyl)-sulfamide;
*N*-[(5-chlorobenzo[*b*]thien-3-yl)methyl]-sulfamide;
*N*-(3-benzofuranylmethyl)-sulfamide;
*N*-[(5-fluorobenzo[b]thien-3-yl)methyl]-sulfamide;
*N*-(1-benzo[*b*]thien-3-ylethyl)-sulfamide;
*N*-(1-naphthalenylmethyl)-sulfamide;
*N*-[(2-methyl-3-benzofuranyl)methyl]-sulfamide;
*N*-[(5-bromobenzo[*b*]thien-3-yl)methyl]-sulfamide;
*N*-[(4-bromobenzo[*b*]thien-3-yl)methyl]-sulfamide;
*N*-[(7-fluorobenzo[*b*]thien-3-yl)methyl]-sulfamide;
*N*-[(1-methyl-1*H*-indol-3-yl)methyl]-sulfamide;
*N*-[(4-trifluoromethylbenzo[*b*]thien-3-yl)methyl]-sulfamide;
*N*-[(4-cyanobenzo[*b*]thien-3-yl)methyl]-sulfamide;
*N*-[(benzo[*b*]thien-3-yl)methyl]-sulfamoylpyrrolidine;
*N*-[(benzo[*b*]thien-3-yl)methyl]-*N*'-ethylsulfamide;
imidazole-1-sulfonic acid [(benzo[*b*]thien-3-yl)methyl]-amide;
and pharmaceutically acceptable salts thereof.

9. A compound as in Claim 8, selected from the group consisting of *N-*(benzo[b]thien-3-ylmethyl)-sulfamide; *N*-[(5-fluorobenzo[b]thien-3-yl)methyl]-sulfamide; and pharmaceutically acceptable salts thereof.

10. A compound selected from the group consisting of *N*-(benzo[*b*]thien-3-ylmethyl)-sulfamide and pharmaceutically acceptable salts thereof.

11. A compound selected from the group consisting of 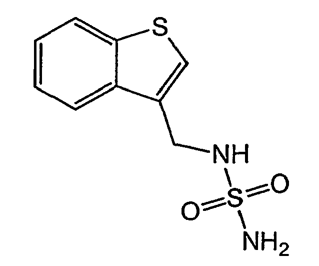 and pharmaceutically acceptable salts thereof.

12. A compound of the formula (I) 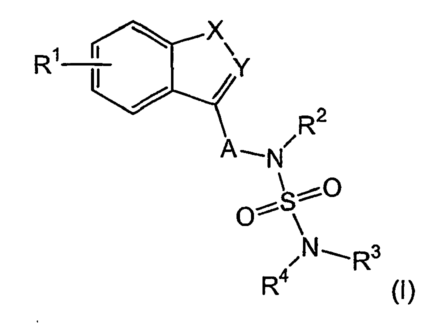 wherein
R¹ is selected from the group consisting of hydrogen, halogen, hydroxy, methoxy, trifluoromethyl, nitro and cyano;
X-Y is selected from the group consisting of -S-CH-, -S-C(CH₃)-, -O-CH-, -O-C(CH₃)-, -N(CH₃)-CH- and -CH=CH-CH-;
A is selected from the group consisting of -CH₂- and -CH(CH₃)-;
R² is selected from the group consisting of hydrogen and methyl;
R³ and R⁴ are each independently selected from the group consisting of hydrogen and methyl;
alternatively, R³ and R⁴ are taken together with the nitrogen atom to which they are bound to form a 5 to 7 membered, saturated, partially unsaturated or aromatic ring structure, optionally containing one to three additional heteroatoms independently selected from the group consisting of O, N and S;
or a pharmaceutically acceptable salt thereof.

13. A pharmaceutical composition comprising a pharmaceutically acceptable carrier and a compound of Claim 1.

14. A pharmaceutical composition made by mixing a compound of Claim 1 and a pharmaceutically acceptable carrier.

15. A process for making a pharmaceutical composition comprising mixing a compound of Claim 1 and a pharmaceutically acceptable carrier.

16. The use of a compound as in Claim 1 for the preparation of a medicament for treating epilepsy or a related disorder selected from seizures, essential tremor or restless limb syndrome, in a subject in need thereof.

17. The use of Claim 16, wherein the related disorder is essential tremor or restless limb syndrome.

18. A compound of any of Claims 1 to 12 for use in therapy.

## Patentansprüche

1. Verbindung der Formel (I) 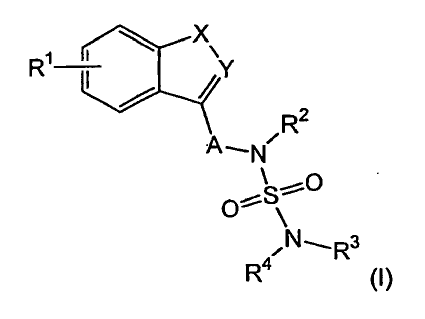 worin
R¹ ausgewählt ist aus der Gruppe, bestehend aus Wasserstoff, Halogen, Hydroxy, Methoxy, Trifluormethyl, Nitro und Cyano;
X-Y ausgewählt ist aus der Gruppe, bestehend aus -S-CH-, -S-C(CH₃)-, -O-CH-, -O-C(CH₃)-, -N(CH₃)-CH- und -CH=CH-CH-;
A ausgewählt ist aus der Gruppe, bestehend aus -CH₂- und -CH(CH₃)-;
R² ausgewählt ist aus der Gruppe, bestehend aus Wasserstoff und Methyl;
R³ und R⁴ jeweils unabhängig ausgewählt sind aus der Gruppe, bestehend aus Wasserstoff und C₁₋₄-Alkyl;
alternativ R³ und R⁴ mit dem Stickstoffatom, an das sie gebunden sind, zusammengenommen sind, um eine 5- bis 7-gliedrige, gesättigte, teilweise ungesättigte oder aromatische Ringstruktur zu bilden, die gegebenenfalls ein bis zwei zusätzliche Heteroatome enthält, die unabhängig ausgewählt sind aus der Gruppe, bestehend aus O, N und S;
oder ein pharmazeutisch verträgliches Salz davon.

2. Verbindung nach Anspruch 1, wobei
R¹ ausgewählt ist aus der Gruppe, bestehend aus Wasserstoff, Halogen, Trifluormethyl, Cyano und Nitro;
X-Y ausgewählt ist aus der Gruppe, bestehend aus -S-CH-, -O-CH-, -O-C(CH₃)-, -N(CH₃)-CH- und -CH=CH-CH-;
A ausgewählt ist aus der Gruppe, bestehend aus -CH₂- und -CH(CH₃)-;
R² ausgewählt ist aus der Gruppe, bestehend aus Wasserstoff und Methyl;
R³ und R⁴ jeweils unabhängig ausgewählt sind aus der Gruppe, bestehend aus Wasserstoff, Methyl und Ethyl;
oder ein pharmazeutisch verträgliches Salz davon.

3. Verbindung nach Anspruch 2, wobei
R¹ ausgewählt ist aus der Gruppe, bestehend aus Wasserstoff, Halogen, Trifluormethyl und Cyano;
X-Y ausgewählt ist aus der Gruppe, bestehend aus -S-CH-, -O-CH-, -O-C(CH₃)-, -N(CH₃)-CH- und -CH=CH-CH-;
A ausgewählt ist aus der Gruppe, bestehend aus -CH₂- und -CH(CH₃)-;
R² Wasserstoff ist;
R³ und R⁴ jeweils unabhängig ausgewählt sind aus der Gruppe, bestehend aus Wasserstoff und Ethyl;
oder ein pharmazeutisch verträgliches Salz davon.

4. Verbindung nach Anspruch 3, wobei
R¹ ausgewählt ist aus der Gruppe, bestehend aus Wasserstoff, 5-Chlor, 5-Fluor, 5-Brom, 4-Brom, 7-Fluor, 5-Trifluormethyl und 5-Cyano;
X-Y ausgewählt ist aus der Gruppe, bestehend aus -S-CH-, -O-CH-, -O-C(CH₃)-, -N(CH₃)-CH- und -CH=CH-CH-;
A ausgewählt ist aus der Gruppe, bestehend aus -CH₂- und -CH(CH₃)-;
R² Wasserstoff ist;
R³ und R⁴ jeweils Wasserstoff sind; alternativ R³ Wasserstoff ist und R⁴ Ethyl ist;
oder ein pharmazeutisch verträgliches Salz davon.

5. Verbindung nach Anspruch 1, wobei
R¹ ausgewählt ist aus der Gruppe, bestehend aus Wasserstoff, Halogen, Trifluormethyl und Cyano;
X-Y ausgewählt ist aus der Gruppe, bestehend aus -S-CH-, -O-CH-, -O-C(CH₃)-, -N(CH₃)-CH- und -CH=CH-CH-;
A ausgewählt ist aus der Gruppe, bestehend aus -CH₂- und -CH(CH₃)-;
R² ausgewählt ist aus der Gruppe, bestehend aus Wasserstoff und Methyl;
R³ und R⁴ mit dem Stickstoffatom, an das sie gebunden sind, zusammengenommen sind, um eine 5- bis 7-gliedrige, gesättigte, teilweise ungesättigte oder aromatische Ringstruktur zu bilden, die gegebenenfalls ein bis zwei zusätzliche Heteroatome enthält, die unabhängig ausgewählt sind aus der Gruppe, bestehend aus O, N und S;
oder ein pharmazeutisch verträgliches Salz davon.

6. Verbindung nach Anspruch 5, wobei
R¹ ausgewählt ist aus der Gruppe, bestehend aus Wasserstoff, Halogen, Trifluormethyl und Cyano;
X-Y ausgewählt ist aus der Gruppe, bestehend aus -S-CH-, -O-CH-, -O-C(CH₃)-, -N(CH₃)-CH- und -CH=CH-CH-;
A ausgewählt ist aus der Gruppe, bestehend aus -CH₂- und -CH(CH₃)-;
R² ausgewählt ist aus der Gruppe, bestehend aus Wasserstoff und Methyl;
R³ und R⁴ mit dem Stickstoffatom, an das sie gebunden sind, zusammengenommen sind, um eine 5- bis 6-gliedrige, gesättigte oder aromatische Ringstruktur zu bilden, die gegebenenfalls ein bis zwei zusätzliche Heteroatome enthält, die unabhängig ausgewählt sind aus der Gruppe, bestehend aus O, N und S;
oder ein pharmazeutisch verträgliches Salz davon.

7. Verbindung nach Anspruch 6, wobei
R¹ Wasserstoff ist;
X-Y -S-CH- ist;
A -CH₂- ist;
R² Wasserstoff ist;
R³ und R⁴ mit dem Stickstoffatom, an das sie gebunden sind, zusammengenommen sind, um eine 5-gliedrige Ringstruktur zu bilden, die ausgewählt ist aus der Gruppe, bestehend aus Pyrrolidinyl und Imidazolyl;
oder ein pharmazeutisch verträgliches Salz davon.

8. Verbindung nach Anspruch 2, ausgewählt aus der Gruppe, bestehend aus
N-(Benzo[*b*]thien-3-ylmethyl)-sulfamid;
N-[(5-Chlorbenzo[*b*]thien-3-yl)methyl]-sulfamid;
N-(3-Benzofuranylmethyl)-sulfamid;
N-[(5-Fluorbenzo[b]thien-3-yl)methyl]-sulfamid;
N-(1-Benzo[*b*]thien-3-ylethyl)-sulfamid;
N-(1-Naphthalinylmethyl)-sulfamid;
N-[(2-Methyl-3-benzofuranyl)methyl]-sulfamid;
N-[(5-Brombenzo[*b*]thien-3-yl)methyl]-sulfamid;
N-[(4-Brombenzo[*b*]thien-3-yl)methyl]-sulfamid;
N-[(7-Fluorbenzo[*b*]thien-3-yl)methyl]-sulfamid;
N-[(1-Methyl-1*H*-indol-3-yl)methyl]-sulfamid;
N-[(4-Trifluormethylbenzo[*b*]thien-3-yl)methyl]-sulfamid;
N-[(4-Cyanobenzo[*b*]thien-3-yl)methyl]-sulfamid;
N-[(Benzo[b]thien-3-yl)methyl]-sulfamoylpyrrolidin;
N-[(Benzo[b]thien-3-yl)methyl]-N'-ethylsulfamid;
Imidazol-1-sulfonsäure[(benzo[*b*]thien-3-yl)methyl]-amid;
und pharmazeutisch verträgliche Salze davon.

9. Verbindung nach Anspruch 8, ausgewählt aus der Gruppe, bestehend aus N-(Benzo[*b*]thien-3-ylmethyl)-sulfamid; N-[(5-Fluorbenzo[*b*]thien-3-yl)methyl]-sulfamid; und pharmazeutisch verträglichen Salzen davon.

10. Verbindung, ausgewählt aus der Gruppe, bestehend aus N-(Benzo[*b*]thien-3-ylmethyl)-sulfamid und pharmazeutisch verträglichen Salzen davon.

11. Verbindung, ausgewählt aus der Gruppe, bestehend aus 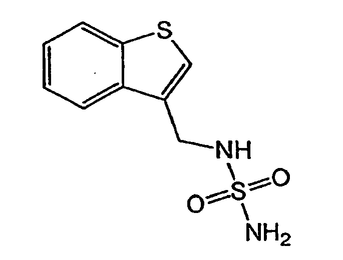 und pharmazeutisch verträglichen Salzen davon.

12. Verbindung der Formel (I) 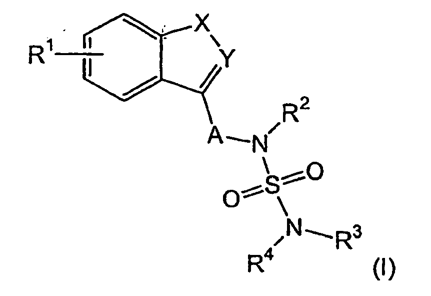 worin
R¹ ausgewählt ist aus der Gruppe, bestehend aus Wasserstoff, Halogen, Hydroxy, Methoxy, Trifluormethyl, Nitro und Cyano;
X-Y ausgewählt ist aus der Gruppe, bestehend aus -S-CH-, -S-C(CH₃)-, -O-CH-, -O-C(CH₃)-, -N(CH₃)-CH- und -CH=CH-CH-;
A ausgewählt ist aus der Gruppe, bestehend aus -CH₂- und -CH(CH₃)-;
R² ausgewählt ist aus der Gruppe, bestehend aus Wasserstoff und Methyl;
R³ und R⁴ jeweils unabhängig ausgewählt sind aus der Gruppe, bestehend aus Wasserstoff und Methyl;
alternativ R³ und R⁴ mit dem Stickstoffatom, an das sie gebunden sind, zusammengenommen sind, um eine 5- bis 7-gliedrige, gesättigte, teilweise ungesättigte oder aromatische Ringstruktur zu bilden, die gegebenenfalls ein bis drei zusätzliche Heteroatome enthält, die unabhängig ausgewählt sind aus der Gruppe, bestehend aus O, N und S;
oder ein pharmazeutisch verträgliches Salz davon.

13. Pharmazeutische Zusammensetzung, die einen pharmazeutisch verträglichen Trägerstoff und eine Verbindung nach Anspruch 1 umfasst.

14. Pharmazeutische Zusammensetzung, hergestellt durch Vermischen einer Verbindung nach Anspruch 1 und eines pharmazeutisch verträglichen Trägerstoffes.

15. Verfahren zur Herstellung einer pharmazeutischen Zusammensetzung, das das Vermischen einer Verbindung nach Anspruch 1 und eines pharmazeutisch verträglichen Trägerstoffes umfasst.

16. Verwendung einer Verbindung nach Anspruch 1 zur Herstellung eines Arzneimittels zur Behandlung von Epilepsie oder einer verwandten Störung, ausgewählt aus epileptischen Anfällen, essentiellem Tremor oder Syndrom der unruhigen Glieder, bei einem Patienten, der derselben bedarf.

17. Verbindung nach Anspruch 16, wobei die verwandte Störung essentieller Tremor oder Syndrom der unruhigen Glieder ist.

18. Verbindung nach einem der Ansprüche 1 bis 12 zur Verwendung in der Therapie.

## Revendications

1. Composé de la formule (I) 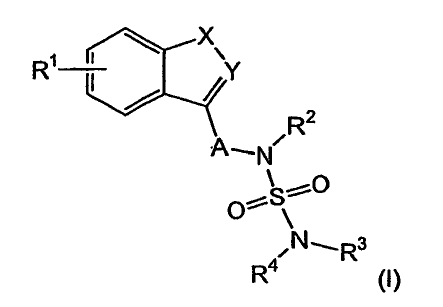 où
R¹ est sélectionné dans le groupe consistant en hydrogène, halogène, hydroxy, méthoxy, trifluorométhyle, nitro et cyano ;
X-Y est sélectionné dans le groupe consistant en -S-CH, -S-C(CH₃)-, -O-CH- , O-C(CH₃)-, -N(CH₃)-CH- et -CH=C_{H}-C_{H}- ;
A est sélectionné dans le groupe consistant en -CH₂- et -CH(CH₃)- ;
R² est sélectionné dans le groupe consistant en hydrogène et méthyle ;
R³ et R⁴ sont chacun indépendamment sélectionnés dans le groupe consistant en hydrogène et alkyle C₁₋₄ ;
alternativement, R³ et R⁴ sont pris ensemble avec l'atome d'azote auquel ils sont liés pour former une structure de cycles de 5 à 7 membres, saturés, partiellement insaturés ou aromatiques contenant facultativement un à deux hétéroatomes additionnels indépendamment sélectionnés dans le groupe consistant en O, N et S ;
ou un sel pharmaceutiquement acceptable de celui-ci.

2. Composé selon la revendication 1, où
R¹ est sélectionné dans le groupe consistant en hydrogène, halogène, trifluorométhyle, cyano et nitro ;
X-Y est sélectionné dans le groupe consistant en -S-CH-, -O-CH-, -O-C(CH₃)-, -N(CH₃)-CH- et -CH=CH-CH- ;
A est sélectionné dans le groupe consistant en -CH₂- et -CH(CH₃)- ;
R² est sélectionné dans le groupe consistant en hydrogène et méthyle ;
R³ et R⁴ sont chacun indépendamment sélectionnés dans le groupe consistant en hydrogène, méthyle et éthyle ;
ou un sel pharmaceutiquement acceptable de celui-ci.

3. Composé selon la revendication 2, où
R¹ est sélectionné dans le groupe consistant en hydrogène, halogène, trifluorométhyle et cyano ;
X-Y est sélectionné dans le groupe consistant en -S-CH-, -O-CH-, -O-C(CH₃)-, -N(CH₃)-CH- et -CH=CH-CH- ;
A est sélectionné dans le groupe consistant en -CH₂- et -CH(CH₃)- ;
R² est hydrogène ;
R³ et R⁴ sont chacun indépendamment sélectionnés dans le groupe consistant en hydrogène et éthyle ;
ou un sel pharmaceutiquement acceptable de celui-ci.

4. Composé selon la revendication 3, où
R¹ est sélectionné dans le groupe consistant en hydrogène, 5-chloro, 5-fluoro, 5-bromo, 4-bromo, 7-fluoro, 5-trifluorométhyle et 5-cyano ;
X-Y est sélectionné dans le groupe consistant en -S-CH-, -O-CH-, -O-C(CH₃)-, -N(CH₃)-CH- et -CH=CH-CH- ;
A est sélectionné dans le groupe consistant en -CH₂- et -CH(CH₃)- ;
R² est hydrogène ;
R³ et R⁴ sont chacun hydrogène ; alternativement R³ est hydrogène et R⁴ est éthyle ;
ou un sel pharmaceutiquement acceptable de celui-ci.

5. Composé selon la revendication 1, où
R¹ est sélectionné dans le groupe consistant en hydrogène, halogène, trifluorométhyle et cyano ;
X-Y est sélectionné dans le groupe consistant en -S-CH-, -O-CH, -O-C(CH₃)-, -N(CH₃)-CH- et -CH=CH-CH- ;
A est sélectionné dans le groupe consistant en -CH₂- et -CH(CH₃)- ;
R² est sélectionné dans le groupe consistant en hydrogène et méthyle ;
R³ et R⁴ sont pris ensemble avec l'atome d'azote auquel ils sont liés pour former une structure de cycles à 5 à 7 membres, saturés, partiellement insaturés ou aromatiques contenant facultativement un à deux hétéroatomes additionnels indépendamment sélectionnés dans le groupe consistant en O, N et S ;
ou un sel pharmaceutiquement acceptable de ceux-ci.

6. Composé selon la revendication 1, où
R¹ est sélectionné dans le groupe consistant en hydrogène, halogène, trichlorométhyle et cyano ;
X-Y est sélectionné dans le groupe consistant en -S-CH-, -O-CH-, -O-C(CH₃)-, -N(CH₃)-CH- et -CH=CH-CH- ;
A est sélectionné dans le groupe consistant en -CH₂- et -CH(CH₃)- ;
R² est sélectionné dans le groupe consistatn en hydrogène et méthyle ;
R³ et R⁴ sont pris ensemble avec l'atome d'azote auquel ils sont liés pour former une structure de cycles à 5 à 6 membres, saturés ou aromatique contenant facultativement un à deux hétéroatomes additionnels indépendamment sélectionnés dans le groupe consistant en O, N et S ;
ou un sel pharmaceutiquement acceptable de ceux-ci.

7. Composé selon la revendication 6, où
R¹ est hydrogène ;
X-Y est -S-CH ;
A est -CH₂- ;
R² est hydrogène ;
R³ et R⁴ sont pris ensemble avec l'atome d'azote auquel ils sont liés pour former une structure de cycles à 5 membres sélectionnés dans le groupe consistant en pyrrolidinyle et imidazolyle ;
ou un sel pharmaceutiquement acceptable de celui-ci.

8. Composé selon la revendication 2, sélectionné dans le groupe consistant en
*N*-(benzo[b]thién-3-ylméthyl)-sulfamide ;
*N*-[(5-chlorobenzo[b]thién-3-yl)méthyl]-sulfamide ;
*N*-(3-benzofuranylméthyl)-sulfamide ;
*N*-[(5-fluorobenzo[b]thién-3-yl)méthyl]-sulfamide ;
*N*-(1-benzo[b]thién-3-yléthyl)-sulfamide ;
*N*-(1-naphalénylméthyl)-sulfamide ;
*N*-[(2-méthyl-3-benzofuranyl)méthyl]-sulfamide ;
*N*-[(5-bromobenzo[b]thién-3-yl)méthyl]-sulfamide ;
*N*-[(4-bromobenzo[b]thién-3-yl)méthyl]-sulfamide ;
*N*-[(7-fluorobenzo[b]thién-3-yl)méthyl]-sulfamide ;
*N*-[(1-méthyl-1H-indol-3-yl)méthyl]-sulfamide ;
*N*-[(trifluorométhylbenzo[b]thién-3-yl)méthyl]-sulfamide ;
*N*-[(4-cyanobenzo[b]thién-3-yl)méthyl]-sulfamide ;
*N*-[(benzo[b]thién-3-yl)méthyl]-sulfamoylpyrrolidine ;
*N*-[(benzo[b]thién-3-yl)méthyl]-*N*-éthylsulfamide ;
Imidazole-1-acide sulfonique [(benzo[b]thién-3-yl)méthyl]-amide ;
et leurs sels pharmaceutiquement acceptables.

9. Composé selon la revendication 8, sélectionné dans le groupe consistant en *N*-(benzo[b]thién-3-ylméthyl)-sulfamide ; *N*-[(5-fluorobenzo[b]thién-3-yl)méthyl]-sulfamide ; et leurs sels pharmaceutiquement acceptables.

10. Composé sélectionné dans le groupe consistant en *N*-(benzo[b]thién-3-ylméthyl)-sulfamide et ses sels pharmaceutiquement acceptables.

11. Composé sélectionné dans le groupe consistant en 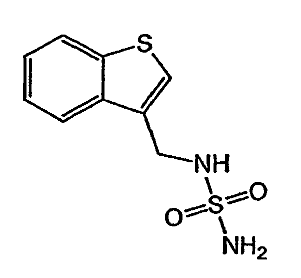 et ses sels pharmaceutiquement acceptables.

12. Composé de la formule (I) 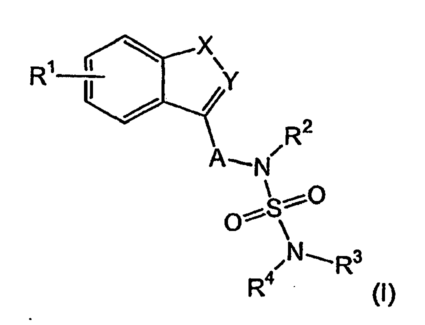 où
R¹ est sélectionné dans le groupe consistant en hydrogène, halogène, hydroxy, méthoxy, trifluorométhyle, nitro et cyano ;
X-Y est sélectionné dans le groupe consistant en -S-CH, -S-C(CH₃)-, -O-CH-O-C(CH₃)-, -N(CH₃)-CH- et -CH=CH-CH- ;
A est sélectionné dans le groupe consistant en -CH₂- et -CH(CH₃)- ;
R² est sélectionné dans le groupe consistant en hydrogène et méthyle ;
R³ et R⁴ sont chacun indépendamment sélectionnés dans le groupe consistant en hydrogène et méthyle ;
alternativement, R³ et R⁴ sont pris ensemble avec l'atome d'azote auquel ils sont liés pour former une structure de cycles de 5 à 7 membres, saturés, partiellement insaturés ou aromatiques contenant facultativement un à trois hétéroatomes additionnels indépendamment sélectionnés dans le groupe consistant en O, N et S ;
ou un sel pharmaceutiquement acceptable de celui-ci.

13. Composition pharmaceutique comprenant un support pharmaceutiquement acceptable et un composé de la revendication 1.

14. Composition pharmaceutique faite en mélangeant un composé de la revendication 1 et un support pharmaceutiquement acceptable.

15. Procédé de production d'une composition pharmaceutique comprenant le mélange d'un composé de la revendication 1 et d'un support pharmaceutiquement acceptable.

16. Utilisation d'un composé selon la revendication 1 pour la préparation d'un médicament pour le traitement de l'épilepsie ou d'un trouble en rapport sélectionné parmi les crises, un tremblement essentiel ou un syndrome des membres sans repos chez un sujet le nécessitant.

17. Utilisation de la revendication 16, où le trouble en rapport est le tremblement essentiel ou le syndrome des membres sans repos.

18. Composé de l'une quelconque des revendications 1 à 12 à utiliser en thérapie.
